# FASCICULE DE BREVET EUROPEEN

(11) **EP 1 392 222 B1**
(45) Date de publication et mention de la délivrance du brevet: **05.09.2007**
(21) Numéro de dépôt: 02747520.1
(22) Date de dépôt: 07.06.2002
(51) Int. Cl.: A61K 8/89, A61K 8/90, A61Q 1/00, A61Q 1/06, A61Q 5/06

(54) **COMPOSITION COSMETIQUE FORMANT APRES APPLICATION UN POLYMERE SUPRAMOLECULAIRE**
KOSMETISCHES MITTEL, DAS NACH ANWENDUNG EIN SUPRAMOLEKULARES POLYMER BILDET
COSMETIC COMPOSITION FORMING AFTER APPLICATION A SUPRAMOLECULAR POLYMER

(30) Priorité: 07.06.2001 FR 0107476
(43) Date de publication de la demande: 03.03.2004
(73) Titulaire: L'ORÉAL, 75008 Paris (FR)
(72) Inventeur: MOUGIN, Nathalie, F-75011 Paris (FR); LIVOREIL, Aude, F-75006 Paris (FR); MONDET, Jean, F-93600 Aulnay sous Bois (FR)
(74) Mandataire: Catherine, Alain
(86) Numéro de dépôt international: PCT/FR2002/001966
(87) Numéro de publication internationale: WO 2002/098377

(56) Documents cités:
- EP-A- 0 868 906
- EP-A- 1 155 687
- WO-A-01/11956
- WO-A-98/14504
- DE-A- 1 595 016
- FR-A- 2 034 760
- FR-A- 2 765 480
- FR-A- 2 772 771
- US-A- 5 919 441
- US-A- 5 958 390
- US-A- 5 998 570
- US-A- 6 051 216

## Description

La présente invention concerne de manière générale une composition cosmétique de soin, et/ou de traitement, et/ou de maquillage des matières kératiniques, présentant une persistance accrue d'au moins un effet cosmétique et/ou de soin apporté après application par la composition, une bonne adhésion après application sur les matières kératiniques, et permettant un démaquillage rapide, total et sélectif.

En particulier, cette composition peut être filmogène, et conduire après application à la filmification de la composition au cours du séchage,

En cosmétique, en effet, on cherche à obtenir un dépôt sur les cheveux, la peau, les cils et les ongles, souvent filmogènes et apportant :
- soit la mise en forme de la coiffure (cheveux) ;
- soit de la couleur (cheveux, maquillage) ;
- soit de la brillance, soit de la brillance et de la couleur (rouge à lèvres, mascaras, eye-liners et vernis à ongle) ;
- soit de la couleur et de la matité (fond de teint), la perte de la matité étant due la plupart du temps à l'évolution de la couleur apportée sous l'effet de la sécrétion du sébum et/ou de la sueur, qui font briller la peau ;
- soit du soin, si le dépôt contient un actif de soin, par exemple hydratant, déodorant, filtres solaires, etc....

On cherche donc pour une persistance de l'effet apporté (couleur, brillance, matité, soin) une grande rémanence du dépôt cosmétique qui doit, en particulier :
- résister aux agressions mécaniques telles que frottements, transferts par contact d'un autre objet ;
- résister à l'eau, à la sueur, aux larmes, à la pluie ; et
- résister au sébum et aux huiles.

Ceci est particulièrement vrai en maquillage pour :
- les rouge à lèvres où l'on recherche la tenue prolongée de la couleur et de la brillance et le non transfert de la couleur ;
- les fonds de teint, fards à paupière et poudres où l'on recherche également la tenue de la couleur apportée, en maintenant le plus longtemps possible la matité de la teinte initiale malgré la sécrétion de sébum et de sueur, ainsi que le non transfert.

En particulier, dans le cas des compositions de maquillage, il serait extrêmement souhaitable de disposer de compositions colorées ayant une constance accrue du coloris, c'est-à-dire dont le coloris n'est pas détérioré ou affadé par les agressions mécaniques, l'eau, la sueur, les larmes, la pluie, le sébum et les huiles, tout en conservant leurs autres propriétés telles que la tenue de la couleur, la brillance, la matité et le soin.

En outre, il serait extrèmement souhaitable que ces compositions, présentant une persistance accrue des effets apportés (notamment couleur, brillance, matité, soin), favorisent l'adhésion sur les matières kératiniques tout en facilitant le démaquillage.

L'homme de l'art connaît l'utilisation de composés filmogènes classiques, tels que les cires, les polymères à liaisons covalentes et les polymères blocs pour la persistance de l'effet cosmétique et/ou de soin.

Ainsi, le brevet EP-0.206.671 décrit une composition cosmétique comprenant un copolymère d'un fluoroalkyl(méth)acrylate et d'un (méth)acrylate d'alkyle linéaire et une huile volatile. Le copolymère confère à la composition cosmétique, lorsqu'elle est appliquée à la peau, une excellente résistance à l'eau et à l'huile, permet un étalement homogène de la composition et conduit à un film d'adhésion satisfaisante et de résistance élevée au frottement. En outre, les copolymères peuvent être formulées en produits solides tels que les sticks ou crayons huileux ayant un toucher extrêmement agréable et la propriété filmogène des copolymères et l'effet de maintien de la condition de maquillage du film obtenu sont tous deux satisfaisants.

Le demande de brevet européen EP-0.815.836 décrit une composition cosmétique comprenant un copolymère de fluoroalkyl(méth)acrylate et une substance huileuse solide qui, après application, présente une excellente brillance et conserve une bonne brillance et couleur sur une période prolongée de temps. Cette composition peut être utilisée comme rouge à lèvres ou mascara.

Lé brevet US 5.948.393 décrit une composition cosmétique de maquillage eau-dans-huile comprenant un matériau colorant dans une phase interne et une résine liposoluble dans une phase externe. La résine liposoluble peut être une fluororésine, une résine de silicone, une résine d'hydrocarbure aromatique, une résine terpènique, du polybutène, du polyisoprène, une résine alkyde et un polymère modifié par la polyvinylpyrrolidone. La composition de maquillage obtenue présente des résistances supérieures à l'eau, à la transpiration, au sébum et aux huiles.

Le brevet US 5.945.108 décrit une composition cosmétique, en particulier de maquillage, qui renferme une poudre hydrofuge et oléofuge obtenue par traitement de la surface de la poudre avec un homopolymère ou copolymère comportant des motifs récurrents (méth)acrylates renfermant un groupe polyfluoroalkyle. Les copolymères (méth)acryliques du document peuvent comporter les combinaisons de monomères suivantes :
- (méth)acrylates renfermant un groupe polyfluoroalkyle et alkyl(méth)acrylate ;
- (méth)acrylates renfermant un groupe polyfluoroalkyle et macromonomères de silicone ; et
- (méth)acrylates renfermant un groupe polyfluoroalkyle, alkyl(méth) acrylate et macromonomère de silicone.

Les compositions cosmétiques préparées par formulation de telles poudres présentent des propriétés hydrofuges et oléofuges et de confort supérieures et empêchent les dégradations du maquillage.

Toutefois, il existe encore le besoin de compositions cosmétiques qui conduisent après application sur les matières kératiniques à des dépôts permettant de concilier la persistance de l'effet cosmétique, et/ou de soin, une bonne adhésion de la composition sur les matières kératiniques et un démaquillage rapide et complet.

Or, la demanderesse a trouvé de manière surprenante qu'une composition cosmétique comprenant un polymère comportant un squelette polymérique comprenant au moins deux motifs de répétition, et au moins deux groupes de jonction fixés sur le squelette polymérique, chaque groupe de jonction étant capable d'interagir avec tout groupe de jonction partenaire via la formation d'au moins 3 liaisons H, avait pour effet d'accroître la persistance d'au moins un effet cosmétique et/ou de soin apporté après application sur les matières kératiniques par la composition.

Par groupe de jonction, on entend, au sens de l'invention, tout groupe fonctionnel comportant des groupes donneurs ou accepteurs de liaisons H, également appelés motifs.

Par groupe de jonction partenaire, on entend, au sens de l'invention tout groupe de jonction d'un polymère selon l'invention pouvant établir des liaisons H avec un ou plusieurs groupes de jonction d'un autre polymère selon l'invention. Les groupes de jonction peuvent être de nature chimique identique ou différente. S'ils sont identiques, ils peuvent alors établir des liaisons H entre eux. S'ils sont différents, ils sont choisis de telle façon qu'ils soient complémentaires vis à vis des interactions H.

La demanderesse a également découvert que cette composition favorisait l'adhésion sur les matières kératiniques. En effet, la kératine comporte des groupes pouvant établir des interactions hydrogène. Par conséquent, l'utilisation dans une composition cosmétique de polymères comportant des groupes pouvant chacun établir des interactions hydrogène fortes et multiples favorise fortement l'adhésion de la composition sur la kératine.

En outre, la demanderesse a aussi découvert que l'utilisation d'un démaquillant sélectif pouvant pénétrer dans le dépôt et rompre sélectivement les interactions établies permet un démaquillage rapide, total et sélectif.

Enfin, la demanderesse a découvert que l'utilisation de tels polymères dans une composition cosmétique conduit, après application de cette composition sur les matières kératiniques, à la formation d'un polymère supramoléculaire.

Par polymère supramoléculaire, on entend, au sens de l'invention, une chaîne ou un réseau polymérique formé de l'assemblage d'un polymère selon l'invention avec au moins un autre polymère selon l'invention, chaque assemblage comprenant au moins une paire de groupe de jonction appariés.

Par paire de groupe de jonction appariés, on entend, au sens de l'invention, deux groupes de jonction appartenant chacun à un polymère selon l'invention reliés ensemble via au moins trois liaisons H.

Lorsque le polymère est formulé en présence de solvants volatils, il est possible d'obtenir la filmification de la composition au cours du séchage, lorsque la structure du polymère le permet.

Par filmification, on entend, au sens de l'invention la formation de réseaux réticulés physiquement se présentant sous forme de films et possédant des propriétés mécaniques analogues à celles de polymères à haut poids moléculaire ou de réseaux réticulés chimiquement. La réticulation physique assurera le maintien et la persistance de l'effet cosmétique et/ou de soin de manière analogue à la réticulation chimique, tout en permettant la réversibilité, c'est à dire la possibilité d'éliminer totalement le dépôt par un démaquillant spécifique, ce que ne permet pas la réticulation chimique.

Il est connu de l'homme du métier d'utiliser, dans des compositions contenant une phase grasse liquide, des polymères comportant des groupes pouvant établir des liaisons hydrogène, pour structurer la phase grasse.

Ainsi, les brevets américains US-5,919,441, US-5,981,680 et US-6,051,216 ainsi que la demande de brevet internationale WO 99/06473 décrivent une composition comprenant une huile de silicone gélifiée par un polyorganosiloxane comportant des groupes pouvant donner des interactions H, notamment des groupes ester, amide, uréthane, urée, thiourée et leurs combinaisons. Cependant, ces polyorganosiloxanes porteurs de groupes à interactions H ne sont utilisés que pour la gélification du milieu à base d'huile de silicone.

De même, le brevet américain US-5,998,570 et la demande de brevet internationale WO/0040216, décrivent des polymères comportant des groupes pouvant établir des liaisons hydrogènes. Chacun de ces groupes ne peut cependant établir qu'une ou deux liaisons H. De tels polymères sont utilisés pour gélifier les huiles hydrocarbonées.

Aucun de ces documents ne concerne une composition cosmétique comprenant un polymère porteur d'au moins deux groupes de jonction capables de faire intervenir chacun au moins trois interactions H. En outre, rien n'est dit dans ces documents concernant les propriétés apportées après application par la composition, telles que la persistance accrue d'au moins un effet cosmétique et/ou de soin, une bonne adhésion de la composition sur les matières kératiniques et la possibilité d'un démaquillage rapide, total et sélectif.

Par ailleurs, la demande internationale WO-98/14504 décrit des polymères comprenant au moins deux groupes, pouvant établir chacun quatre interactions H, réversibles avec la température. Ces polymères sont principalement utilisés pour leur facilité de mise en oeuvre à chaud, notamment à une température où les molécules se comportent individuellement les unes par rapport aux autres, sans être engagées dans des liaisons. Rien n'est dit concernant une utilisation cosmétique de tels polymères et ses problèmes spécifiques.

L'invention a donc pour objet une composition cosmétique de soin, et/ou de traitement et/ou de maquillage des matières kératiniques permettant de pallier ces inconvénients.

De façon plus précise, l'invention a pour objet une composition cosmétique de soin, et/ou de traitement et/ou de maquillage des matières kératiniques, qui comprend, dans un milieu physiologiquement acceptable une quantité efficace d'au moins un polymère linéaire, ramifié, ou cyclique, ou dendrimère, comportant :
- un squelette polymérique -POL- comprenant au moins deux motifs de répétition, et
- au moins deux groupes de jonction (A) fixés sur le squelette polymérique et capables d'établir des liaisons H avec un ou plusieurs groupes de jonction partenaires, de nature chimique identique ou différente, chaque appartement de deux groupes de jonction faisant intervenir au moins trois liaisons H, les groupes de jonction (A) étant choisis parmi : adénine, guanine, cytidine, thymine, ptérine, uréidopyrimidone, mélamine, acide cyanurique, maléïmide, phtalhydrazide, isoguanine, glycoluril, uracil, acylaminopyridine, de préférence monoacylamino pyridine et Bis(acylamino) pyridine, acylaminotriazine, de préférence mono-acyl 2,4-diamino-s-triazine et Bis-acyl 2,4-diamino-s-triazine, pyridine/phénol, urazole, glutarimide, acide urazoyl benzoïque, succinimide, 2,4-diamino-s-triazine, uréidotriazine.

Par squelette polymérique, on entend, au sens de l'invention un polymère comportant au moins deux motifs de répétition covalents.

Les deux groupes de jonction (A) peuvent être latéraux, dans la chaîne polymérique ou aux extrémités de la chaîne polymérique.

Les groupes de jonction (A) comportent des groupes donneurs et/ou accepteurs de liaisons H, également appelés motifs, et choisis parmi les fonctions chimiques suivantes : 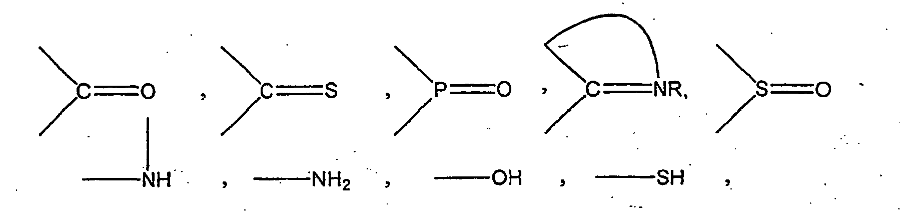 où R représente :
- un atome d'hydrogène, ou
- un groupement aryle, ou
- un groupement aralkyle, c'est-à-dire aryle substitué par une chaîne hydrocarbonée saturée, linéaire ou ramifiée, comprenant 1 à 40 atomes de carbone, ou
- une chaîne hydrocarbonée saturée, linéaire, ramifiée ou cyclique, comprenant 1 à 40 atomes de carbone, et/ou comprenant éventuellement un ou plusieurs hétéroatomes choisi parmi O, S et N, et/ou éventuellement substituée par un ou plusieurs atomes de fluor et/ou radicaux hydroxy.

De manière avantageuse, ces polymères ont un squelette polymérique ayant un degré de polymérisation allant de 2 à 10000, et de préférence de 10 à 5000.

### Structure du polymère selon l'invention

On symbolisera les polymères de l'invention par :

A-POL-A

où :
- A désigne un groupe de jonction
- POL désigne le squelette polymérique.

Les polymères selon l'invention peuvent présenter différentes structures.
de manière générale, ils peuvent être :
- linéaires ou ramifiés et fonctionnalisés uniquement aux extrémités :

   A-POL-A
- linéaires ou ramifiés et comportant au moins deux-groupes dans la chaîne :

   A-POL-A-A-

   ou

   A-A-POL-A-

   (ici un groupe à une extrémité)
- linéaires et portant les groupes A en ramification latérales : 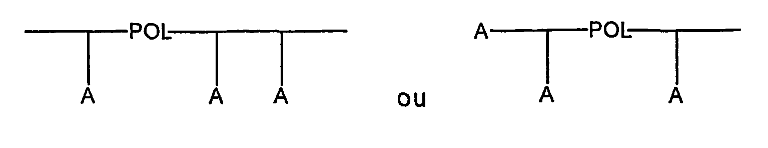
- hyperbranchés ou dendrimères, les groupes A étant aux extrémités : 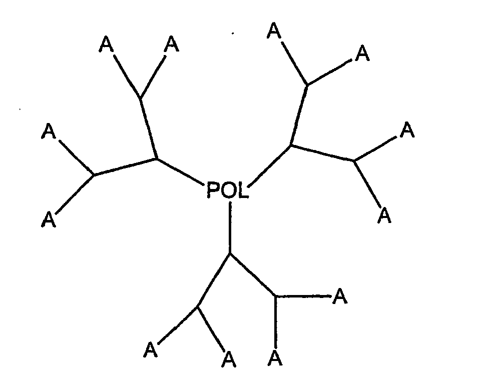
- ou en étoile :

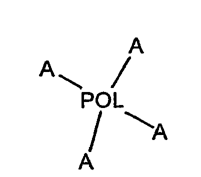

Les structures préférées des polymères selon l'invention sont :

### 1. Les polymères linéaires et α, ω fonctionnels :

A-POL-A

(seulement deux groupes)

L'utilisation de tels polymères dans une composition cosmétique conduit après application de la composition sur les matières kératiniques, à la formation d'un polymère supramoléculaire sous forme d'une longue chaîne polymère (de poids moléculaire élevé). Les polymères selon l'invention s'ajoutent bout à bout par l'appariement de chacun de leurs groupes de jonction avec un groupe de jonction (A), identique chimiquement ou autocomplémentaire, d'un autre polymère :

A-POL-A A-POL-A A-POL-A A-PLO-A

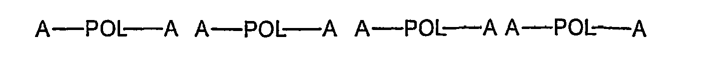

### 2. Les polymères tri ou multifonctionnels :

### trifonctionnels: multifonctionnels à groupes A latéraux

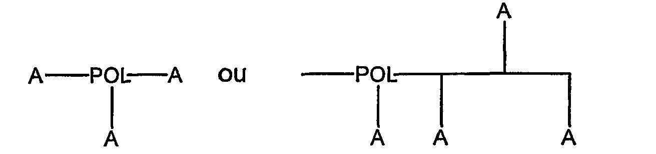

Les polymères tri- ou multifonctionnels, utilisés seuls ou en mélange avec des polymères difonctionnels, peuvent conduire, après application de la composition sur les matières kératiniques, à la formation d'un polymère supramoléculaire constitué par un réseau réticulé physiquement, se présentant sous forme de film, et ayant une très bonne résistance mécanique et aux solvants. De tels films peuvent être éliminés de manière sélective (démaquillage) par un solvant contenant un rupteur d'interactions H et pouvant pénétrer dans le dépôt. Les réseaux ainsi formés peuvent être schématisés de la manière suivante :

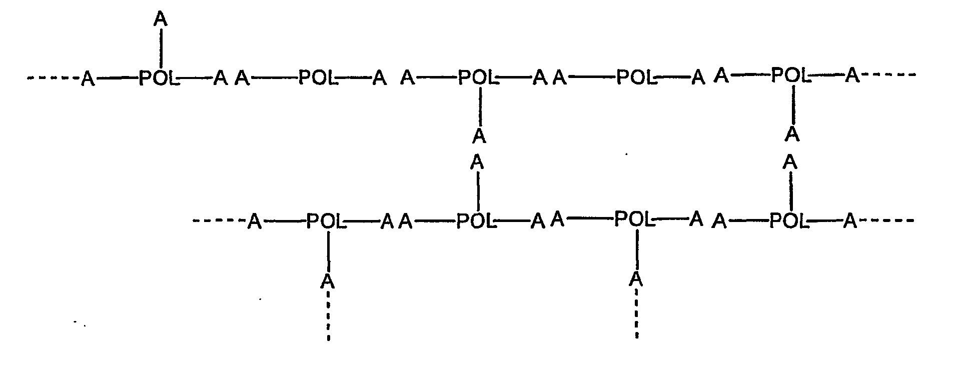

### 3. Les polymères en étoile, hyperbranchés ou ramifiés (cas particulier des multifonctionnels)

Utilisés en mélange avec des polymères difonctionnels, ces polymères peuvent conduire, après application de la composition sur les matières kératiniques, à la formation d'un polymère supramoléculaire constitué par un réseau réticulé physiquement, identique aux réseaux obtenus à partir de polymères tri- ou multifonctionnels, mais avec une meilleure probabilité de réticulation, ce qui permet d'utiliser une quantité de polymère plus faible pour une même efficacité de réticulation. Les réseaux ainsi formés peuvent être schématisés de la manière suivante :

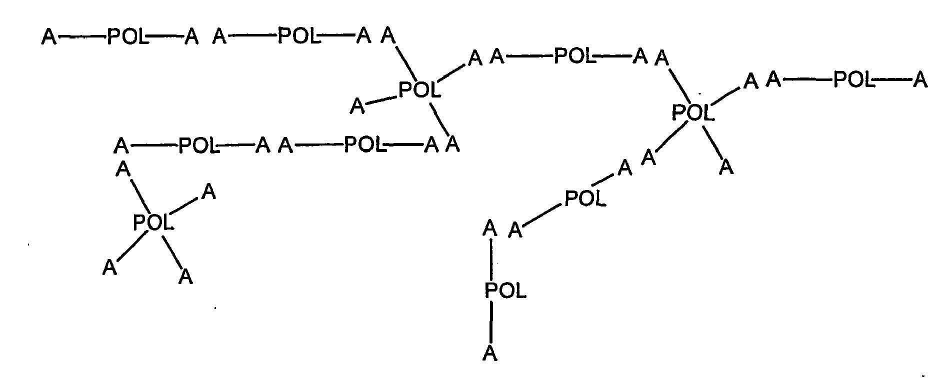

### 4. Les mélanges de polymères à groupes de jonction (A) identiques mais dont les squelettes polymériques sont de nature chimique différente

L'utilisation, dans une composition cosmétique, de mélanges de polymères à groupes de jonction A identiques ou complémentaires mais dont les squelettes polymériques sont de nature chimique différente peut conduire, après application de la composition sur les matières kératiniques, à la formation d'assemblages de type copolymère, mais avec des liaisons physiques entre les motifs au lieu de liaisons covalentes.

Ainsi, on obtiendrait, après dépôt d'une composition comportant deux polymères α,ω bifonctionnels (respectivement de structure polymérique -POL₁- et -POL₂-) un polymère supramoléculaire symbolisé par :

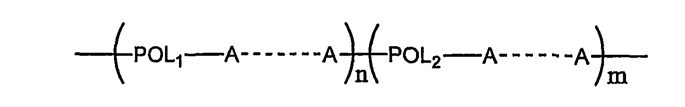

Les enchaînements des polymères dans le polymère supramoléculaire sont aléatoires dans le cas présent, et sont équivalents à des assemblages statistiques.

Il est cependant possible d'obtenir des polymères supramoléculaires équivalents à des copolymères alternés, séquencés, multiséquencés ou greffés, de la même façon qu'en polycondensation covalente.

Selon l'invention, les polymères supramoléculaires sont de préférence statistiques et obtenus par combinaison de squelettes polymériques -POL₁- et -POL₂- tels que :
- -POL1- est hydrophobe, et -POL₂- est hydrophile, pour régler la balance hydrophile/hydrophobe du dépôt, sa résistance à l'eau, à la sueur, au sébum, aux huiles, etc ;
- le mélange des polymères A-POL₁-A et A-POL₂-A est au départ soluble ou véhiculable dans des solvants préétablis ;
- le dépôt final a des propriétés mécaniques préétablies, par exemple par combinaison d'un squelette polymérique -POL₁- rigide avec d'un squelette polymérique -POL₂- flexible

### Groupe de jonction A

Les groupes de jonction (A) comportent obligatoirement une ou plusieurs fonctions choisis parmi: 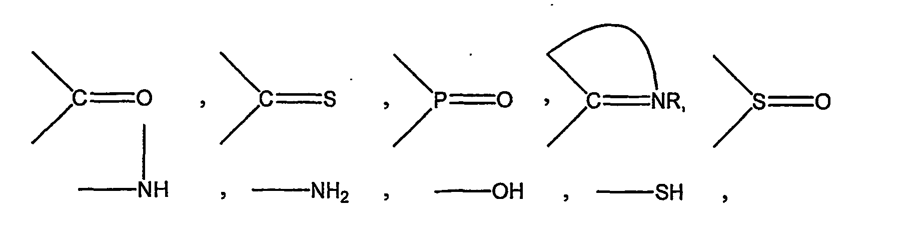 où R représente :
- un atome d'hydrogène, ou
- un groupement aryle, ou
- un groupement aralkyle, c'est-à-dire aryle substitué par une chaîne hydrocarbonée saturée, linéaire ou ramifiée, comprenant 1 à 40 atomes de carbone ; ou
- une chaîne hydrocarbonée saturée, linéaire, ramifiée ou cyclique, comprenant 1 à 40 atomes de carbone, et/ou comprenant éventuellement un ou plusieurs hétéroatomes choisi parmi O, S et N, et/ou éventuellement substituée par un ou plusieurs atomes de fluor et/ou radicaux hydroxy.

De préférence, les groupes de jonction A comportent des cycles en 5, 6 atomes (cycles aromatiques ou hétérocycles insaturés), très souvent constitués d'atomes de C et N et avec des doubles liaisons conjuguées pour stabiliser et diriger les interactions H.

Les groupes de jonction (A) auto-complémentaires sont choisis parmi les groupes : adénine, guanine, cytidine, thymine, ptériné, ureïdopyrimidone, mélamine, acide cyanurique, maléïmide, phtalhydrazide, isoguanine, glycoluril, uracil, acylaminopyridine, acylaminotriazine, pyridine/phénol, urazole, glutarimide, acide urazoyl benzoïque.

Chaque groupe partenaire (A₁) doit pouvoir établir des liaisons H avec un ou plusieurs groupes partenaires, identiques (A₁) ou différents (A₂), de telle sorte que chaque appariement de deux groupes partenaires se fasse par formation d'au moins trois liaisons H, de préférence quatre liaisons H.

### a) Exemple de groupes (A₁) identiques pouvant établir entre eux au moins trois liaisons H : les groupes uréïdopyrimidone

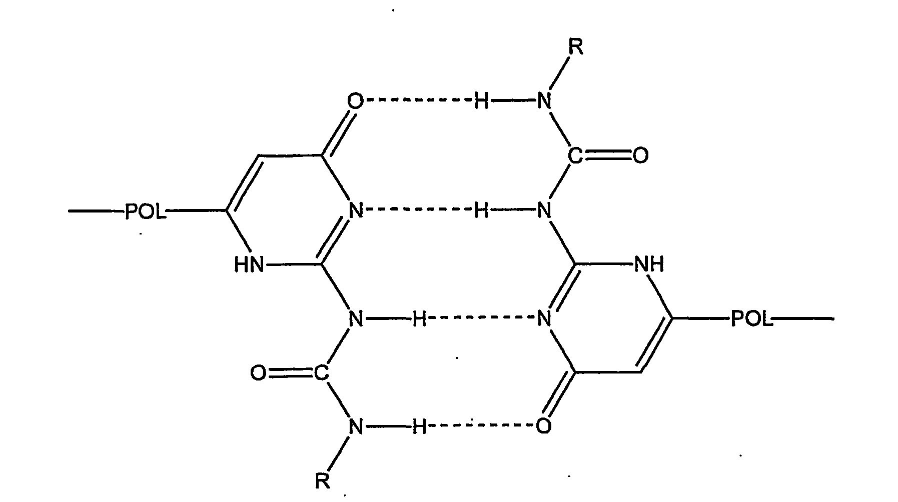

où R désigne H ou un groupe alkyle linéaire ou ramifié, en C₁-C₄₀, pouvant contenir un ou plusieurs atomes tels que O, S, N, P, F, Si, pouvant comporter des insaturations conjuguées ou non conjuguées,
ou bien R désigne un groupe cycloalkyle en C₄-C₈, ou un groupe aryle, ou un groupe alcoxy en C₁-C₄₀ ou un groupe arylcoxy, ou un groupe ester d'alkyle.

Ces groupes interagissent avec eux-mêmes en établissant entre eux quatre liaisons H.

### b) Exemple de groupes (A₁) et (A₂) de nature chimique différente, mais complémentaire vis-à-vis de l'établissement d'au moins trois liaisons H : A₁ = mélamine et A₂ = acide cyanurique

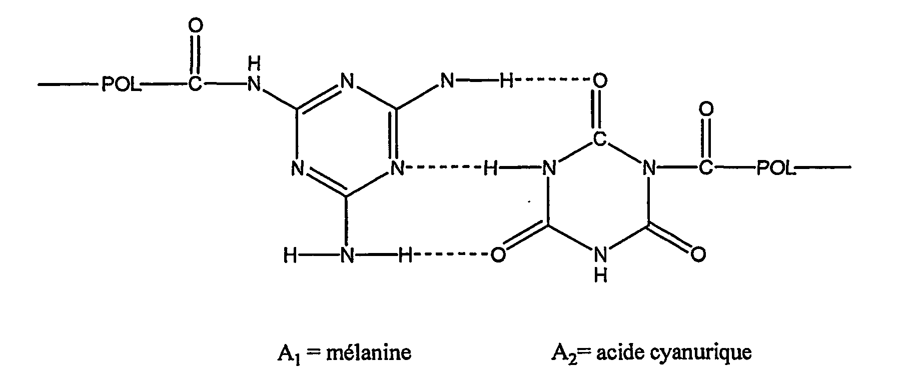

Parmi ou en complément des groupes de jonction (A) cités, on préférera les groupes de jonction suivants :

### 1.Bis(acylamino)pyridine de structure (l) :

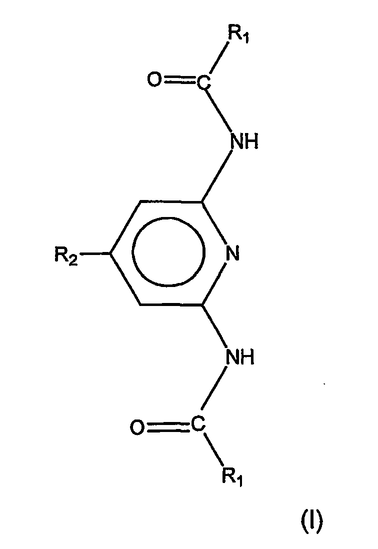

où
- R₁ et R₂ désignent H ou un groupe alkyle linéaire ou ramifié, en C₁-C₄₀, pouvant contenir un ou plusieurs atomes tels que O, S, N, P, F, Si, pouvant comporter des insaturations conjuguées ou non conjuguées, ou bien, R₁ et R₂ désignent un groupe cycloalkyle en C₄-C₈, ou un groupe aryle, ou un groupe alkoxy en C₁-C₄₀, ou un groupe aryloxy, ou un groupe ester d'alkyle ;
- l'accroche du squelette polymérique -POL- peut se faire en R₁ ou R₂,

Ce groupe de jonction (A) peut interagir avec lui-même, donnant alors quatre interactions H, mais il interagit souvent avec un groupe complémentaire et donne alors trois liaisons H.

### groupes complémentaires du groupe Bis(acylamino)pyridine

- ***uracil*** de structure (II) : 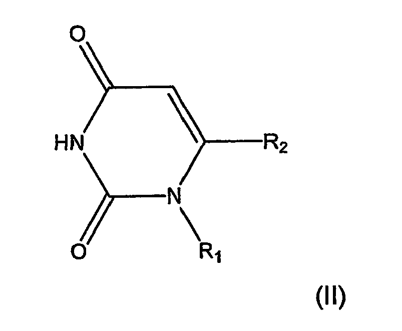 où :
- R₁ et R₂ ont la même signification que précédemment,
- le squelette polymérique -POL- est fixé en R₁ ou R₂.
- ***succinimide*** de structure (III) : 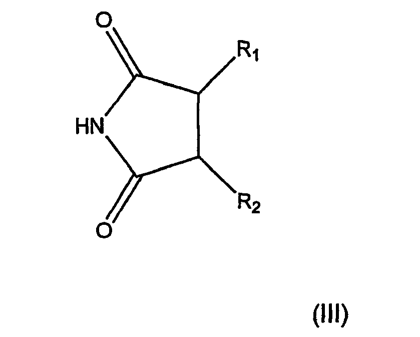 où :
- R₁ et R₂ ont la même signification que précédemment,
- le squelette polymérique -POL- est fixé en R₁ ou R₂.
- *thymine (substitué ou non)* de structure (IV) : 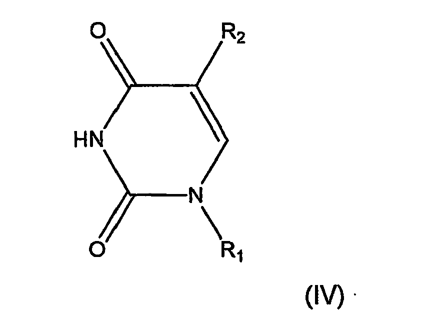 où :
- R₁ et R₂ ont la même signification que précédemment,
- le squelette polymérique -POL- est fixé en R₁ ou R₂.
- ***glutarimide*** de structure (V) : 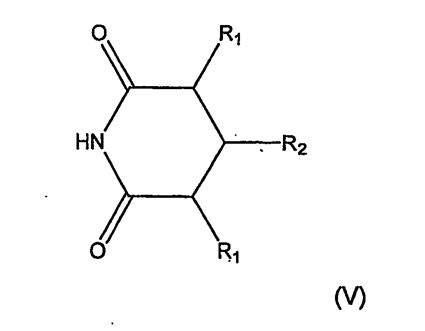 où :
- R₁ et R₂ ont la même signification que précédemment,
- le squelette polymérique -POL- est fixé en R₁ ou R₂.
- ***acide cyanurique*** de structure (VI) : 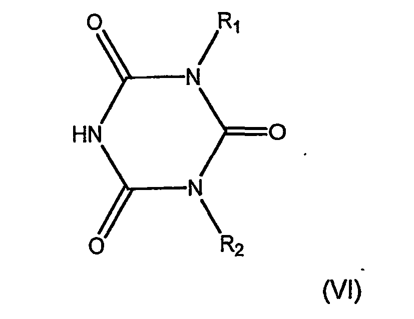 où :
- R₁ et R₂ ont la même signification que précédemment,
- le squelette polymérique -POL- est fixé en R₁ ou R₂.

### 2. Uréïdo pyrimidone de structure (VII):

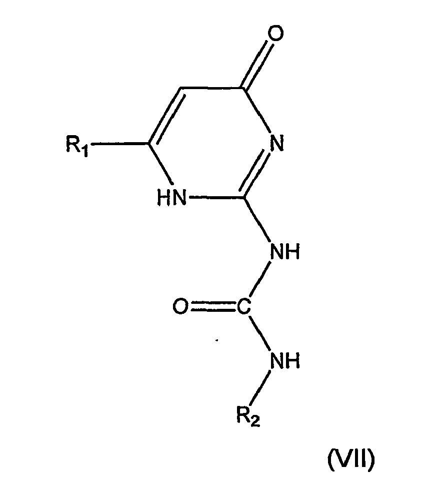

où :
- R₁ et R₂ même signification que précédemment,
- le squelette polymérique -POL- est fixé en R₁ ou R₂.

Ces groupes de jonction interagissent avec eux mêmes en établissant entre eux quatre liaisons H.

### 3. Bis acyl de 2,4-diamino-s-triazine de structure (VIII):

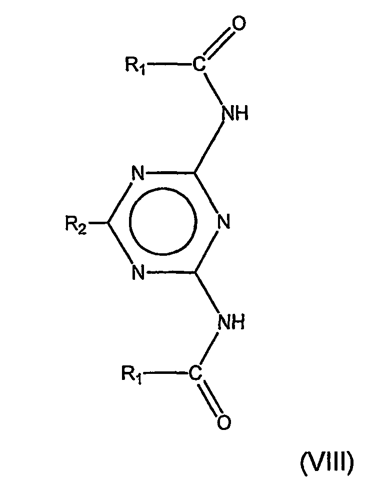

où :
- R₁ et R₂ ont la même signification que précédemment,
- le squelette polymérique -POL- est fixé en R₁ ou R₂.

Ces groupes de jonction interagissent avec eux-mêmes en établissant entre eux quatre liaisons H.

### 4. Uréïdo triazine de structure (lX) :

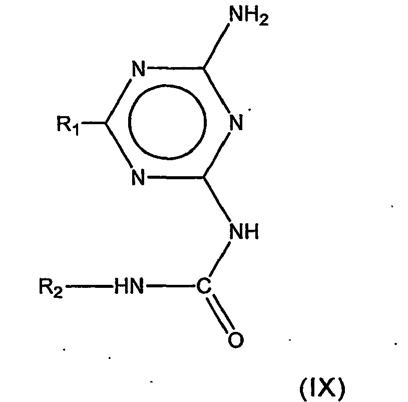

où :
- R₁ et R₂ ont la même signification que précédemment,
- le squelette polymérique -POL- est fixé en R₁ ou R₂.

Ces groupes de jonction interagissent avec eux-mêmes en établissant entre eux quatre liaisons H.

### 5. Phtalhydrazide de structure (X) :

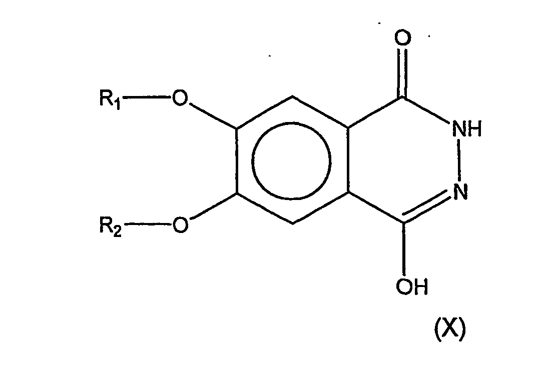

où :
- R₁ et R₂ ont la même signification que précédemment,
- le squelette polymérique -POL- est fixé en R₁ ou R₂.

Ces groupes de jonction interagissent avec eux-mêmes en établissant entre eux quatre liaisons H.

### 6.Acide Urazoyl benzoïque de structure (Xl) :

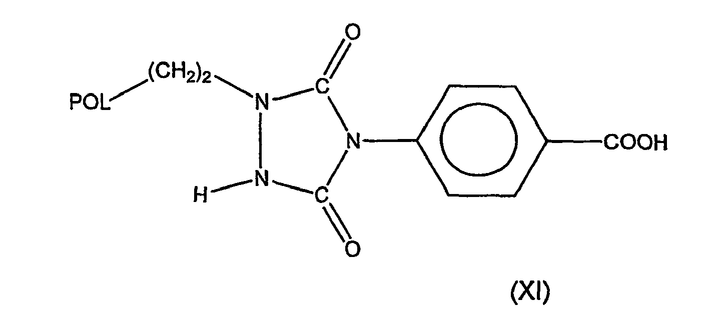

où :
- R₁ et R₂ ont la même signification que précédemment,
- le squelette polymérique -POL- est fixé en R₁ ou R₂.

Ces groupes de jonction interagissent avec eux-mêmes en établissant entre eux quatre liaisons H.

### 7. 2,4-diamino-s-triazine de structure (XII) :

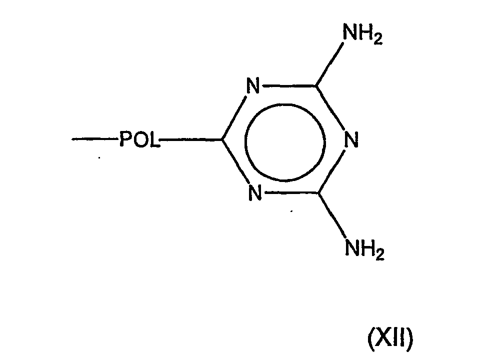

dont les groupes complémentaires peuvent être choisis parmi les groupes uracil, succinimide, thymine, glutarimide et acide cyanurique.

### 8. Monoacylamino pyridine de structure (XIII) :

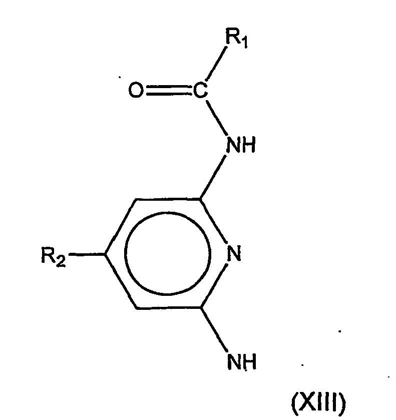

où :
- R₁ et R₂ ont lamême signification que précédemment,
- le squelette polymérique -POL- est fixé en R₁ ou R₂, et
- dont les groupes complémentaires peuvent être choisis parmi les groupes uracil, succinimide, thymine, glutarimide et acide cyanurique.

### 9. Monoacyl de 2,4-diamino-s-triazine de structure (XIV) :

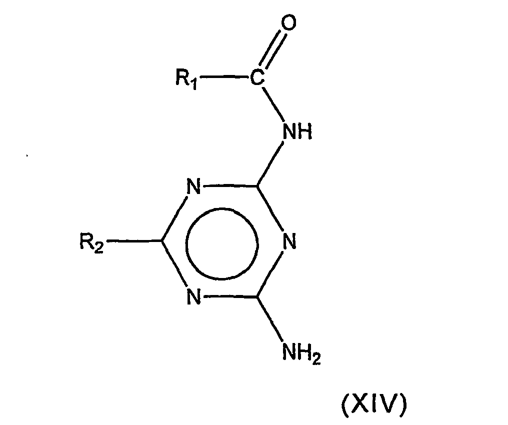

où :
- R₁ et R₂ ont lamême signification que précédemment,
- le squelette polymérique -POL- est fixé en R₁ ou R₂, et
- dont les groupes complémentaires peuvent être choisis parmi les groupes uracil, succinimide, thymine, glutarimide et acide cyanurique.

### 10. Mélamine de structure (XV) :

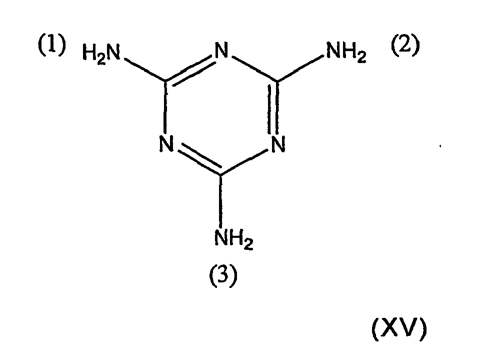

où :
- le squelette polymérique -POL- est fixé en (1), (2) ou (3) par réaction avec 1 motif NH₂, et dont
- les groupes complémentaires peuvent être choisis parmi les groupes uracil, succinimide, thymine, glutaramide et acide cyanurique.

De manière préférée, les groupes de jonction selon l'invention sont capables d'établir au moins quatre liaisons H avec un même groupe jonction partenaire.

On peut citer, à titre d'exemple de groupes de jonction capables d'établir au moins quatre liaisons H avec eux mêmes, les groupes suivants :
- uréïdo-pyrimidone ;
- Bis acyl de 2,4-diamino-3-triazine ;
- uréidotriazine ;
- phtalhydrazide ;
- acide urazoyl benzoïque ;

### Squelette polymérique -POL-

Les squelettes polymériques selon l'invention peuvent être de toute nature chimique et doivent posséder au moins deux motifs répétitifs.

Ils peuvent être obtenus par toutes les techniques de polymérisation (radicalaire, ionique, transfert de groupes, catalyse Ziegler ou métallocène, etc. pour ceux issus de la polymérisation de monomères insaturés) ou de polycondensation. Ils peuvent être issus de dérivés naturels ou naturels modifiés chimiquement.

Le nombre de motifs de répétition ou degré de polymérisation, du squelette polymérique selon l'invention varie généralement de 2 à 100.

De préférence, le degré de polymérisation varie de 10 à 500, pour éviter la cristallisation du polymère selon l'invention et permettre, que la composition selon l'invention ait des propriétés filmogènes après application.

De manière préférée, les squelettes polymériques -POL- ne portent pas d'autres groupes pouvant établir des liaisons H avec les groupes de jonction (A). En d'autres termes, ils ne portent pas de groupes susceptibles d'entrer en compétition avec les groupes de jonction (A) pour l'établissement de liaisons H, ceci afin d'éviter de gêner ou d'affaiblir la formation du polymère supramoléculaire.

A titre de squelettes polymériques selon l'invention, on peut citer avantageusement les polydiènes, les polyesters, les polycarbonates, les polyacétals, les polyoxyalkylènes, les polythioéthers, les perfluoropolyéthers, les polyoléfines, les polyorganosiloxanes, lés polymères vinyliques, les poly(meth)acryliques, les dérivés cellulosiques, les dérivés polysaccharides, notamment les éthers et les esters.

Pour fixer par covalence les groupes de jonction (A) sur les squelettes polymériques -POL-, on partira, de préférence, de radicaux -POL- portant au moins deux groupes fonctionnels réactifs X porteurs d'hydrogènes labiles, X désignant -OH, -NH₂, -NHR, -SH etc....

On citera en particulier:

### 1. Polydiènes, de préférence hydrogénés, à extrémités hydroxyles et polyoléfines à extrémités hydroxyles

Les polydiènes, de préférence hydrogénés, à extrémités hydroxyles et polyoléfines à extrémités hydroxyles sont des squelettes polymériques préférés selon l'invention.

Ces polydiènes à extrémités hydroxyles sont définis par exemple dans le brevet FR-2-782.723 d'ELF ATOCHEM. Ils sont choisis dans le groupe comprenant les homo et copolymères de polybutadiène, de polyisoprène et de poly(1,3-pentadiène). Ce sont des oligomères de une masse moléculaire moyenne en nombre inférieure à 7000, et de préférence de 1000 à 5000, présentant une fonctionnalité en extrémités hydroxyles de 1,8 à 3 et de préférence voisine de 2.

Ces polydiènes sont utilisés, de préférence, hydrogénés.

On citera en particulier les polybutadiènes hydroxylés commercialisés par la société ELF ATOCHEM sous les marques POLY BD R-45HT et POLY BD R-20 LM, qui seront utilisés de préférence hydrogénés.

On peut également utiliser des polyoléfines, homopolymères ou copolymères, à extrémités α,ω hydroxyles tels que:
- les oligomères de polyisobutylène à extrémités α,ω hydroxyles.
- les copolymères commercialisés par la société Mitsubishi sous la marque POLYTAIL avec, en particulier, ceux répondant à la formule (XVI) :

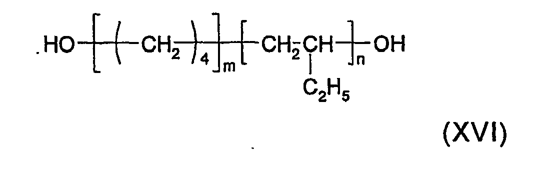

### 2. Polyesters initialement à extrémités α,ω -OH

Les polyesters initialement à extrémité α,ω -OH, également appelés polyesters-polyols, sont aussi des squelettes polymériques -POL- préférés de l'invention.

Les polyesters polyols selon l'invention peuvent être obtenus par réaction entre
- au moins un alcool polyhydrique, choisi parmi l'éthylène glycol, le propylène glycol, le diéthylène-glycol, le néopentyl-glycol, le 1,4-butanediol, le furane diméthanol, le cyclohexane diméthanol, le glycérol, le triméthylpropane, le pentaerythritol et leurs mélanges, et
- au moins un acide polycarboxylique, de préférence un acide dicarboxylique, ou un dérivé, notamment un diester, choisi parmi les acides succinique, glutamique, adipique et leurs diméthyl esters, et l'anhydride phtalique.

Le polyester polyol selon l'invention peut aussi être obtenu par polymérisation d'une lactone, par exemple la caprolactone et d'un polyol.

Toutefois, les polyesters polyols préférés selon l'invention sont les polyesters polyols obtenus par condensation avec un acide gras dimère et/ou un dimère-diol, ainsi que les polyesters polyols obtenus par réaction avec des huiles hydrocarbonées naturelles ou synthétiques portant deux à trois groupes hydroxyles ou époxydes.

### 2.1. Polyester polyol obtenu par condensation avec un acide gras dimère et/ou dimère-diol

Les acides gras dimères sont définis dans les brevets américains US-5.998.570, US-3,157,681 et US-5,411,729, répondant respectivement aux formules (XVII), (XVIII) et (XIX) :

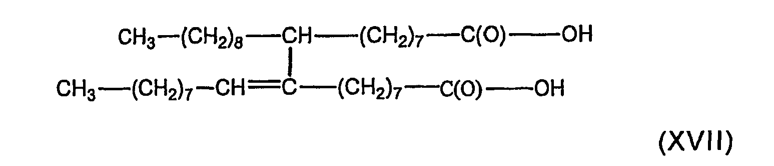

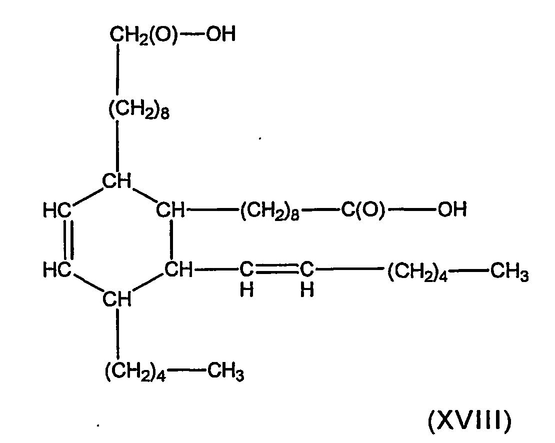

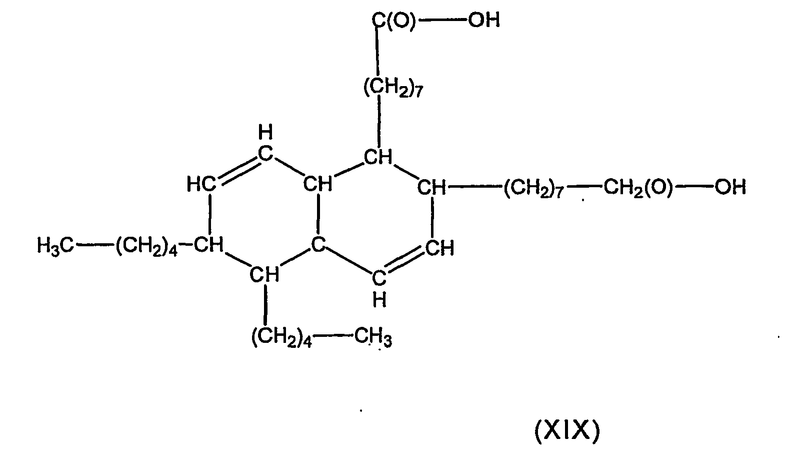

Ils sont notamment commercialisés sous les dénominations :
- UNIDYME® par la société UNION CAMP, et
- PRIPOL (par exemple PRIPOL 1013) par la société UNICHEMA.

Les dimères-diols sont ceux définis dans l'article de R. HÖFER, European Coating Journal, Mars 2000, pages 26-37. Ils possèdent les mêmes structures chimiques que les acides gras dimères correspondants, seules les fonctionnalités changent.

Comme indiqué dans l'article de R. HÖFER, European Coating Journal, Mars 2000, pages 26-37, la transformation des acides gras dimères en dimères diols peut être faite:
- soit par hydrogénation d'esters méthyliques des acides gras dimères,
- soit par dimérisation directe de l'alcool oléique.

On citera, en particulier, les dimères diols vendus par la société COGNIS sous les noms commerciaux de SOVERMOL 908 (à 97,5% en pureté de dimère) et de SOVERMOL 650 NS (à 68% en pureté de dimère).

### 2.2. Polyester polyol obtenus par réaction avec des huiles hydrocarbonées naturelles ou synthétiques portant deux à trois groupes hydroxyles (ou époxydes)

Les huiles préférées sont, bien entendu, les huiles portant deux groupes hydroxyles par chaîne comme les monoglycérides de structure (XX) ou (XXI) : 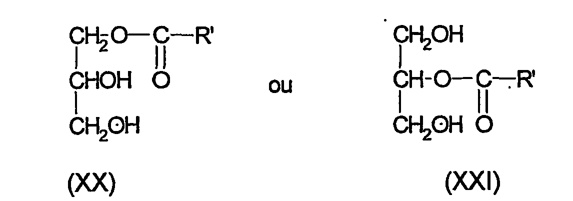 où R' est une chaîne alkyle linéaire ou ramifiée, comme par exemple le monostéarate de glycérol.

De tels monoesters de glycérol correspondent, par exemple, aux composés 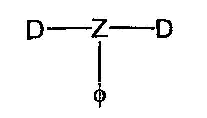 où
- D désigne -O-, et

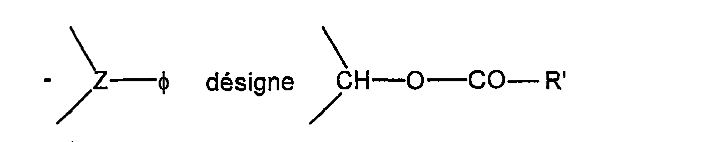

On peut également citer les huiles naturelles (Vernonia) ou synthétiques comportant des groupes époxydes.

### 3. Polycarbonates initialement à extrémités α, ω -OH.

Les squelettes polymériques selon l'invention peuvent également être avantageusement choisis parmi les polycarbonates à extrémités α,ω -OH.

Les polycarbonate à extrémités α,ω -OH peuvent être obtenus par la réaction entre un diol pouvant être choisi parmi le 1,3-propanediol, le 1,4-butanediol, le 1,6-hexanediol, le diéthyèneglycol, et le tétraéthylèneglycol, et un diarylcarbonate comme par exemple un diphénylcarbonate, ou du phosgène.

### 4. Polyalkylène oxydes initialement à extrémités α,ω -OH.

Comme squelette polymérique selon l'invention, on peut également utiliser avantageusement les polyalkylène oxydes à extrémités α, ω - OH.

Les polyalkylène oxydes selon l'invention peuvent être obtenus par polymérisation d'oxydes cycliques, notamment choisis parmi l'oxyde d'éthylène, l'oxyde de propylène et le tétrahydrofurane, ou par addition d'un ou plusieurs oxydes cycliques à des initiateurs polyfonctionnels, notamment choisis parmi l'eau, l'éthylène glycol, le diéthylène glycol, le cyclohexane diéthanol, le glycérol, le triméthyolpropane, le pentaerythritol, et le disphénol A.

Les polyoxypropylène diols et triols, les poly(oxyéthylène-oxopropylène) diols et triols, obtenus par addition simultanée ou séquentielle d'oxydes d'éthylène ou de propylène, glycols à des initiateurs appropriés, sont des polyalkylène oxydes préférés selon l'invention.

De même, les polytétraméthylène glycols obtenus par polymérisation du tétra hydrofuranne; et les polyalkylène oxydes oligomères porteurs d'au moins deux groupes amines aux extrémités de chaîne POE sont des polyalkylène oxydes particulièrement préférés selon l'invention.

On citera également les polyalkylène oxydes oligomères porteurs d'au moins deux groupes amines aux extrémités de chaîne POE, tels que les diamines de polyalkylène oxydes JEFFAMINE® commercialisés par la société TEXACO.

### 5. Polythioéthers initialement à extrémités α, ω-OH.

Il est également possible d'utiliser comme squelette polymérique selon l'invention un polythioéther à extrémité α, ω-OH.

Les polythioéthers utilisés selon l'invention peuvent être obtenus par condensation du thiodiglycol seul ou avec d'autres glycols, ou des acides dicarboxyliques, du formaldéhyde, des aminoalcools ou des acides aminocarboxyliques.

### 6. Polyacétals initialement à extrémités α,ω -OH.

On peut également utiliser un squelette polymérique choisi parmi les polyacétals initialement à extrémité α, ω -OH.

Comme polyacétals utilisables selon l'invention, on citera les polyacétals obtenus par réaction entre
- au moins un glycol, notamment choisi parmi le diéthylèneglycol, le triméthylèneglycol et l'hexanediol, et
- le formaldéhyde.
   On peut également utiliser les polyacétals obtenus par polymérisation d'acétals cycliques.

### 7. Polyorganosiloxanes initialement porteurs d'au moins deux groupes réactifs tels que -OH, -NH₂, -NHR, -SH, et

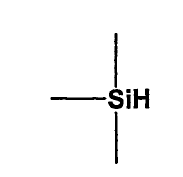

Les polyorganosiloxanes initialement porteurs d'au moins deux groupes réactifs choisis parmi les groupes -OH, -NH₂, -NHR (où R est tel que défini précédemment), -SH, et 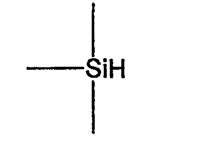 sont également des squelettes polymériques utilisables selon l'invention.

Selon un mode de réalisation particulier de l'invention, on utilisera un polyorganosiloxane α,ω-téléchélique, à extrémités -OH, -NH₂ et - NHR, de structure (XXII) : 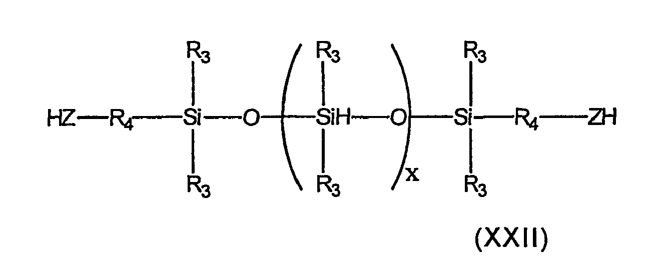 où :
- x désigne un nombre entier de 0 à 100,
- Z représente -O-, ou -NH-, ou -NR-,
- R est tel que défini précédemment,
- R₃ est un groupe choisi parmi les chaînes alkyles ou alcoxy en C₁-C₄₀, linéaires ou ramifiées (avec de préférence -CH₃, -C₂H₅, n-C₃H₇ et iso-C₃H₇), les groupes phényle, éventuellement substitués par 1 à 3 groupes méthyle ou éthyle, les chaînes polyorganosiloxane, les groupes fluoroalkyle en C₁-C₁₂, linéaires
ou ramifiés, et les groupes fluoroalcoxyéthylène en C₁-C_{12,} linéaires ou ramifiés,
- R⁴ est choisi parmi les groupes alkyles divalents en C₁-C₆₀, les groupes oxyalkylènes en C₁-C₆₀ et contenant de 0 à 3 motifs d'oxyde éthylène, et leurs mélanges, et dans lequel chaque atome directement relié à un groupe hydroxyle est un atome de carbone.

On utilisera de préférence les polyorganosiloxanes pour lesquels R₄ est un radical divalent alkylène C₁-C₁₀ où x est un nombre entier tel que la masse moléculaire moyenne en nombre du polysiloxane varie de 300 à 10000.

De manière très préférée, on utilisera des polydiméthylsiloxanes de structure (XXIII) : 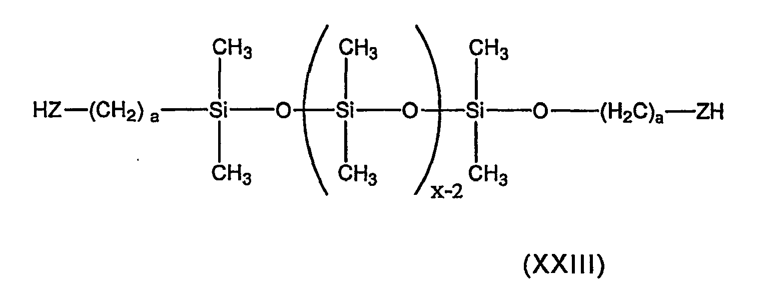 où :
- a désigne un entier allant de 1 à 10, et
- Z est tel que défini ci-dessus.

De tels oligomères α,ω difonctionnels sont notamment commercialisés par la société GOLDSCHMIDT sous les dénominations :
- TEGOMER H-Si 2111 avec x tel que le poids moléculaire moyen en nombre PM est de 700
   (avec Z désignant -O-),
- TEGOMER H-Si 2311 avec x tel que le poids moléculaire moyen en nombre PM est de 2200
   (avec Z désignant -O-),
- TEGOMER A-Si 2122 avec x = 10
   (avec Z désignant -R-NH(R')).

A titre d'exemples d'oligomères α,ω difonctionnels, on peut également citer les oligomères commercialisés par la société SHIN-ETSU sous les dénominations :
- X-22-161AS, X-22-161A, X-22-161B, X-22-161C (avec Z désignant -NH-),
- X-22-160AS, KF-6001, KF-6002, KF-6003 et x-22-4015
   (avec Z désignant -0-),

On peut également citer à titre de polyorganosiloxanes α, ω téléchéliques préférés les polyorganosiloxane pour lesquels R₄ est avantageusement un groupe oxyalkylène, comprenant des motifs oxyde de butylène, ou des motifs oxyde de propylène, ou de 0 à 3 motifs d'oxyde d'éthylène.

On utilisera de préférence les polyorganosiloxanes de structure (XXIV) : 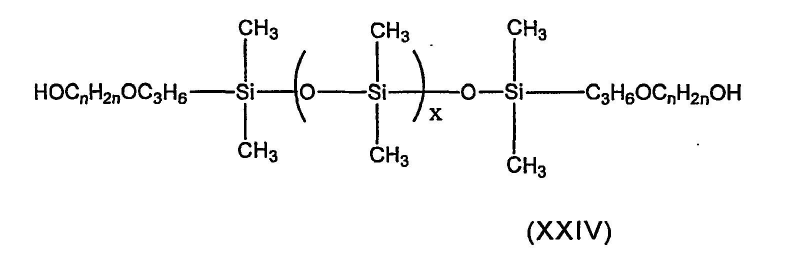 où :
- n varie de 2 à 6 indépendamment, et
- x est un entier de 2 à 100.

A titre d'exemple de tels polyorganosiloxanes, on peut notamment citer les polyorganosiloxanes de structure (XXV) : 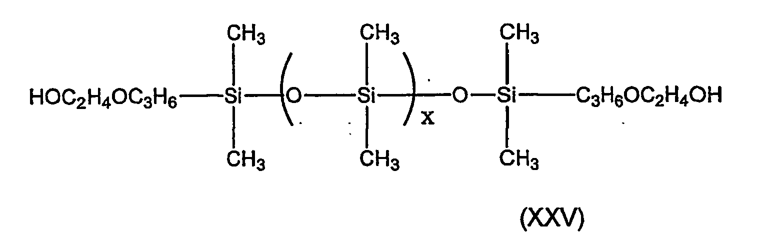 où x est un entier de 2 à 100.

Ces polyorganosiloxanes sont commercialisés sous la dénomination SILAPLANE FM-4425 par la société CHISSO AMERICA, Inc.

Selon un autre mode de réalisation de l'invention, on utilisera des polyorganosiloxanes à greffons polyalkylène oxydes terminés par des groupes -OH (greffons POE ou greffons POE-PPO: diméthicone copolyols habituelles), de structure répondant à la formule (XXVI) : 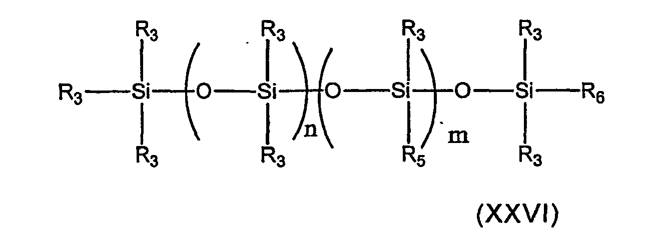 où :
- R₃ a la même signification qu'à la formule (XXII);
- R₅ est un groupe polyalkylène oxyde monovalent avec une terminaison -OH,
- R₆ étant identique à R₃ ou à R₅.

Selon un troisième mode de réalisation de l'invention, on utilisera des polyorganosiloxanes à groupes amines latéraux et/ou terminaux, de structure répondant à la formule (XXVII) : 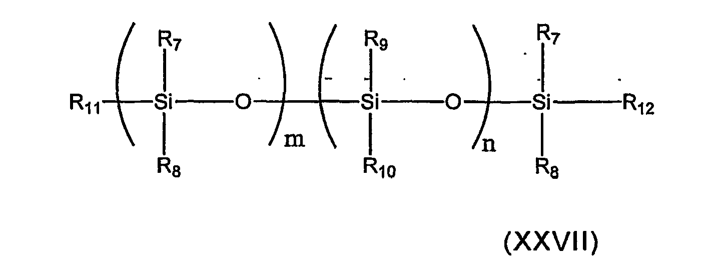 où :
- les groupes représentés par les symboles R₇ et R₉ peuvent être identiques ou différents, et sont choisis parmi les groupes alkyle comportant de 1 à 22 atomes de carbone, phényle, naphtyle ou polyoxyalkylène,
- au moins l'un des groupes représentés par les symboles R₁₀ et R₁₂ est un groupe de structure H₂N-(-R₁₃-NH-)ₛ-R₁₄, où R₁₃ et R₁₄ représentent chacun un groupe alkylène comportant de 1 à 6 atomes de carbone et s vaut 0 ou 1,
- les autres groupes peuvent être identiques ou différents, et sont des groupes alkyle comportant de 1 à 22 atomes de carbone, ou des groupes phényle, ou des groupes naphtyle ou des groupes polyoxyalkylène, et
- m et n représentent chacun un nombre valent au moins 1.

Selon un quatrième mode de réalisation, on utilisera des polyorganosiloxanes à groupes thiols-SH latéraux et/ou terminaux.

Selon un cinquième mode de réalisation, on utilisera des polyorganosiloxanes à groupes latéraux et/ou terminaux hydrogénosilanes de structure générale répondant à la formule (XXVIII) ou (XXIX) : 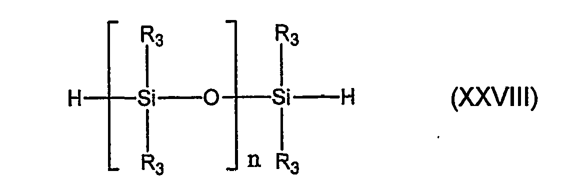 ou 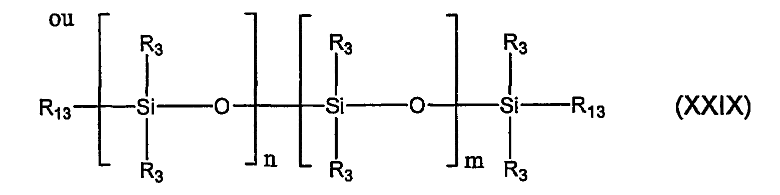 où
- R₃ est tel que défini précédemment (XXII) ;
- R₁₃ représente soit R₃, soit H.

De tels polyorganosiloxanes ne peuvent être utilisées que pour la fixation de groupes de jonction (A) porteurs d'une double liaison (met)allylique.

A titre d'exemple de tels polyorganosiloxanes, on peut citer les polyorganosiloxanes à groupes latéraux et/ou terminaux hydrogénosilanes commercialisés par la société ABCR.

### 8. Perfluoropolyéthers initialement à extrémités α,ω -OH.

Les squelettes polymériques selon l'invention peuvent également être avantageusement choisis parmi les perfluoropolyéthers à extrémités et/ou groupes latéraux hydroxyles, et de préférence les perfluoro polyéther-diols de structure répondant à la formule (XXX) :

HO-Q-Rf-O-(CᵢF₂ᵢO)ⱼ-(C_{y}F_{2y}O)ₖ(C_{z}F_{2z}O)ₚ-Rf-Q'-OH (XXX)

dans laquelle :
- les groupes oxyperfluoroalkylène -(CⱼF₂ⱼO)-, (C_{y}F_{2y}O)- et -(C_{z}F_{2z}O)-sont répartis aléatoirement, ou sont groupés en bloc dans une chaîne,
   - i est un entier de 1 à 10,
   - j est un entier de 0 à 100,
   - k est un entier de 1 à 100,
   - p est un entier de 0 à 100,
   - y est un entier de 1 à 10,
   - z est un entier de 1 à 10,
   - chaque groupe Rf est indépendamment choisi parmi les radicaux divalents perfluoroalkyle comprenant de 1 à 20 atomes de carbone,
   - chaque groupe Q est indépendamment choisi parmi les groupes -C₆H₄-, -C₆H₃Cl-, -C₂H₄OCH₂- et -C_{b}H_{2b}-, et
   - chaque groupe Q' est indépendamment choisi parmi les groupes -C₆H₄-, -C₆H₃Cl-, -C₂H₄OCH₂- et -C_{b}H_{2b}-, et
   - b est un entier de 1 à 20.

De tels perfluoroéthers diols sont par exemple ceux définis dans la demande internationale WO 98/44015 et dans la publication de T. TEMTCHENKO et al., XXVIth International Conference in Organic Coatings, 3-7 July 2000, VOULIAGMENI (ATHENS), Book of Conf., page 357-65.

Ils sont commercialisés par la société AUSIMONT sous les dénominations :
- FLUOROBASE Z 1030, correspondant à k=j=6 ;
- FLUOROBASE Z, correspondant à un rapport k/j = ¼ et un poids moléculaire moyen en nombre (PM) de 1000 à 4000, et de préférence de 500 à 700, avec en particulier un pourcentage pondéral en OH de 4,8 à 6,8%;
- FOMBLIN HC/OH 1000, de poids moléculaire moyen en nombre égal à1036, avec une fonctionnalité en extrémités hydroxyles de 2, et de structure répondant à la formule (XXXI) :

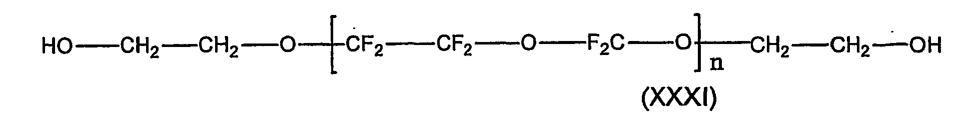

### 9. Oligomères ou polymères vinyliques ou (met)acryliques initialement à groupes réactifs -OH, -NH₂, -NHR, -SH.

Selon l'invention, il est également possible d'utiliser comme squelette polymérique des oligomères ou polymères vinyliques ou (met)acryliques initialement à groupes réactifs -OH, -NH₂, -NHR (où R est tel que défini précédemment), ou -SH.

De tels polymères sont généralement choisis parmi les homo ou copolymères à groupes latéraux -OH, -NH₂, -NHR (où R est tel que défini précédemment).

Ce sont de préférence des oligomères de poids moléculaire moyen en nombre inférieur à 10000, obtenus par copolymérisation de un ou plusieurs monomères vinyliques, allyliques, oléfines, vinyléther, acides ou esters ou amides (meth)acryliques, avec au moins un monomère co-réactif porteur d'au moins un groupe choisi parmi -OH, -NH₂ et -NHR.

A titre d'exemple, on peut citer les homo et copolymères obtenus par polymérisation avec :
- un (méth)acrylate d'hydroxyalkyle, tel que l'acrylate de 2-hydroxyéthyle, ou
- l'alcool vinylique (obtenu par hydrolyse des motifs acétate de vinyle du polymère), ou
- l'alcool allylique,
- la vinylamine, ou
- l'allylamine.

Mais il est également possible d'utiliser les oligomères (homo et co) porteurs d'extrémités réactives α,ω -OH.

La polymérisation de monomères métacryliques (acide, esters ou amides) en présence d'agent de transfert 2-mercaptoéthanol (HS-CH₂-CH₂-OH) conduit à des oligomères fonctionnalisées à chaque extrémités α,ω par un groupe hydroxyéthylsulfure : 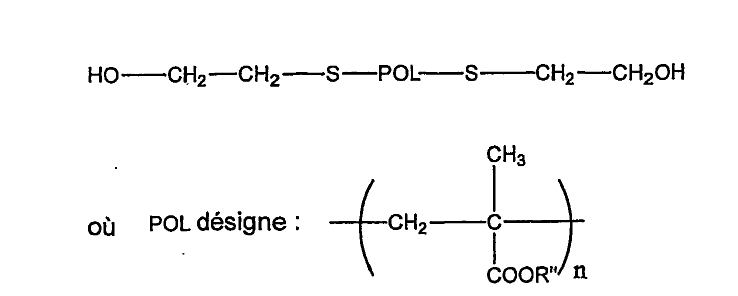 où R" représente un groupe choisi parmi les groupes groupes alkyle, aryle, aralkyle, alcényle, alcoxy, alcoxyalkyle, alcoxyaryle ou phényle.

Cette polymérisation est décrite dans la publication de G. REUSMANN, Eur Coat. J., 9, 52-53, 56, 58 (1999) également N. KEBELKAMP A,, Farbe Lack, 105, (2), 24-26, 28-29 (1999) décrivant la synthèse de tels α, ω polyméthacrylates-diols et leur utilisation pour la réalisation de polyuréthanes.

### 10. Dérivés cellulosiques

Le squelette polymérique POL selon l'invention peut également être choisi parmi les dérivés cellulosiques.

Parmi eux, on peut citer les éthers hydroxyalkyléthers et alkyléthers de cellulose (ou guar, etc...), les esters cellulose (tels que les acétates, propionates, butyrates et esters mixtes), et la nitrocellulose.

### 11. Di, tri, tétra et plus généralement polysaccharides ou leurs dérivés,

Le squelette polymérique POL selon l'invention peut aussi être choisi parmi les di, tri, tétra et plus généralement polysaccharides ou leurs dérivés, notamment éthers et esters.

### 12. hyperbranchés ou dendrimères, initialement à extrémités réactives du type -OH, -NH₂, -NHR et -SH.

Enfin, le squelette polymérique POL selon l'invention peut être choisis parmi les hyperbranchés ou dendrimères, initialement à extrémités réactives du type -OH, -NH₂, -NHR et -SH.

Les dendrimères (du grec dendron = arbre) sont des molécules polymères "arborescentes" c'est-à-dire très ramifiées inventées par D.A. Tomalia et son équipe au début des années 90 (Donald A. Tomalia et al., Angewandte Chemie, lnt. Engl. Ed., vol 29, n° 2, pages 238-175). Il s'agit de structures moléculaires construites autour d'un motif central généralement polyvalent. Autour de ce motif central, sont enchaînés, en couches concentriques et selon une structure parfaitement déterminée, des motifs ramifiés d'allongement de chaîne donnant ainsi naissance à des macromolécules symétriques, monodispersées ayant une structure chimique et stéréochimiques bien définie. Des dendrimères de type polyaminoamine sont commercialisés par exemple sous la dénomination STARBUST® par la société DENDRITECH.

Les polymères hyperramifiés sont des polycondensats, généralement de type polyester, polyamide ou polyéthylèneimine, obtenus à partir de monomères multifonctionnels, qui ont une structure arborescente similaire à celle des dendrimères mais beaucoup moins régulière que celle-ci (voir par exemple les demandes de brevet internationales WO-A-93/17060 et WO-96/12754).

La société PERSTORP commercialise sous la dénomination BOLTORN® des polyesters hyperramifiées. On trouvera sous la dénomination COMBURST® de la société DENDRITECH des polyéthylèneimines hyperramifiées et des hyperbranchés polyesteramides à extrémités de chaîne hydroxyles commercialisés sous le nom HYBRANE® par la société DSM.

Comme déjà indiqué, après fonctionnalisation de ces dendrimères ou hyperbranchés, en extrémités par fixation des groupes de jonction -(A), on les utilisera plutôt en mélange avec d'autres polymères selon l'invention A-POL-A n'ayant que deux groupes (A) par molécule, de façon à réaliser des dépôts réticulés, après application et séchage de la composition cosmétique.

### Fixation des groupes de jonction (A) sur le squelette polymérique POL

Les réactions chimiques de fixation citées ici ne sont pas limitatives mais seulement données à titre d'illustration.

Selon un premier mode de réalisation de l'invention, les groupes de jonction sont fixés sur des squelettes polymériques POL comportant des groupes à hydrogène labile tels que -OH, -NH₂, -NHR (où R est tel que défini précédemment) ou -SH, via la fonctionnalisation du groupe de jonction (A) par un isocyanate. Cette réaction comporte les étapes suivantes:
- fonctionnalisation du groupe A par un isocyanate selon le schéma réactionnel: 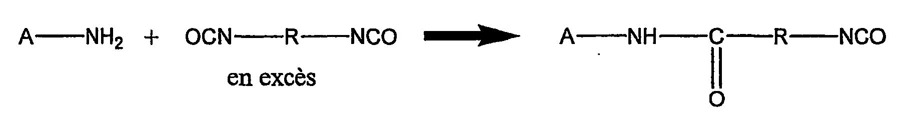
- puis réaction avec un squelette polymérique POL comportant au moins deux groupes à hydrogène labile tel que -OH, -NH₂, ou -SH.

A titre d'exemple, on peut citer la réaction de fixation d'un groupe uréidopyrinidone, comme par exemple le 6-méthylisocytosine, sur un squelette polymérique POL de structure HO-POL-OH ou H₂N-POL-NH₂ :

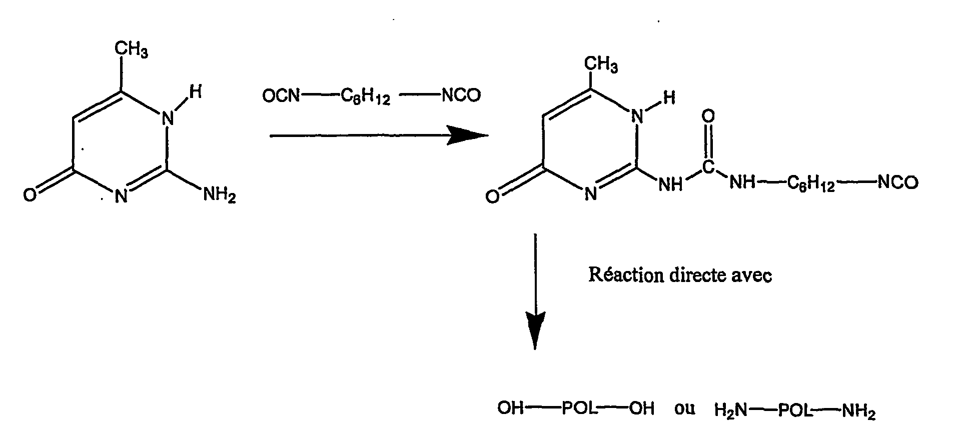

Cette réaction est décrite plus précisément dans la publication de B.J.B. FOLMER, Adv. Mater, 12, 874-78 (2000).

Il est également possible de faire la réaction inverse en préfonctionnalisant le squelette polymérique à groupe à hydrogène labile tels que -OH, NH₂, -NHR (où R est tel que défini précédemment), ou -SH par un diisocyanate.

A titre d'exemple, on peut citer la réaction d'un squelette polymérique POL avec un diisocyanate suivant :

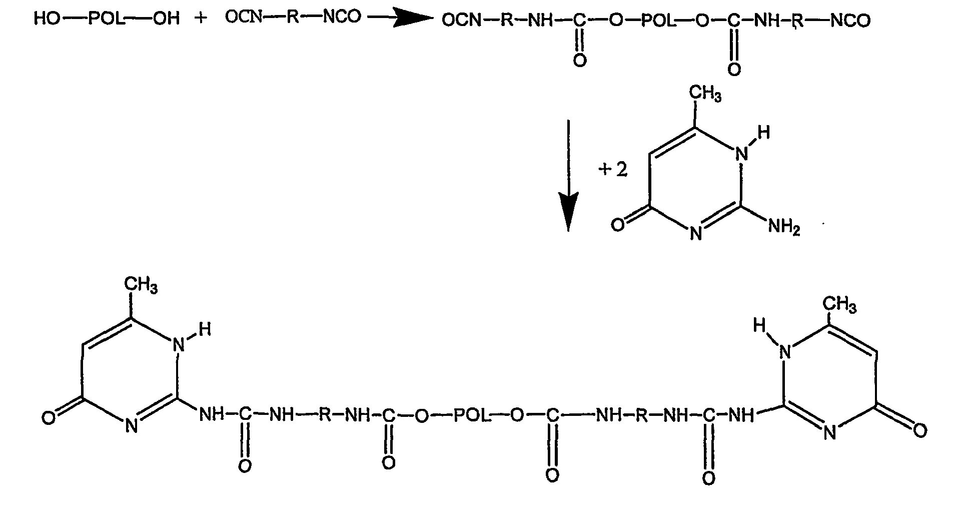

Cette réaction est également décrite dans la publication de B.J.B. FOLNER, Adv. Mater, 12, 874-78(2000). Cette publication donne également les conditions de fixation des groupes uréidopyridimidone, pour l'une ou l'autre des deux voies décrites ci-dessus, sur des squelettes polymériques POL de nature :
- polyoxyalkylène (par exemple : HO-POE/PPO-OH),
- polyester (par exemple : polyadipate de butyle à extrémité -OH),
- polycarbonate (par exemple : polycarbonate d'hexyle) à extrémité α, ω-OH),
- copoly(éthylène/butylène) à extrémités α,ω -OH

Ces deux voies de fixation des groupes uréidopyrimidones peuvent être transposées à tous les squelettes polymériques -POL-selon l'invention comportant deux ou plusieurs groupes -OH, -NH, - NHR, et -SH.

Les conditions de fixation des groupes 6-méthylisocytosine, via l'isophorone diisocyanate sur des copolymères blocs POE/PPO à extrémités -OH sont détaillées dans la publication de R.F.M. LANGE, J. Polymer Sci : Part A, Polym. Chem., 37 3657-70 (1999) ainsi que dans la demande internationale WO-98/14504.

Dans une variante, les groupes de jonction (A) sont fixés sur les polyorganosiloxanes par hydrolysation. Cette réaction comporte les étapes suivantes :
- fonctionnalisation de (A) par un groupe allylique -CH₂-CH=CH₂ par la synthèse directe → CH₂=CH-CH₂-(A) ;
- puis réaction avec un organosiloxane porteur de deux ou plusieurs groupes hydrogénosilanes -SᵢH suivant :

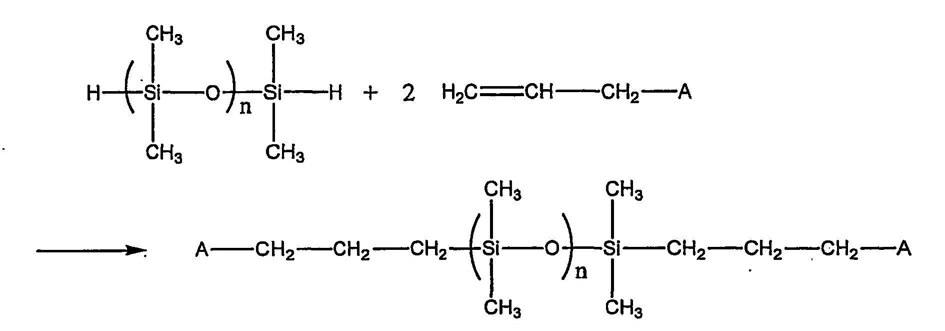

A titre d'exemple, on peut citer la réaction de d'hydrosilylation décrite dans la publication de R.P.S. SIVBESMA, Science, 278, 1601-04 (1997) :

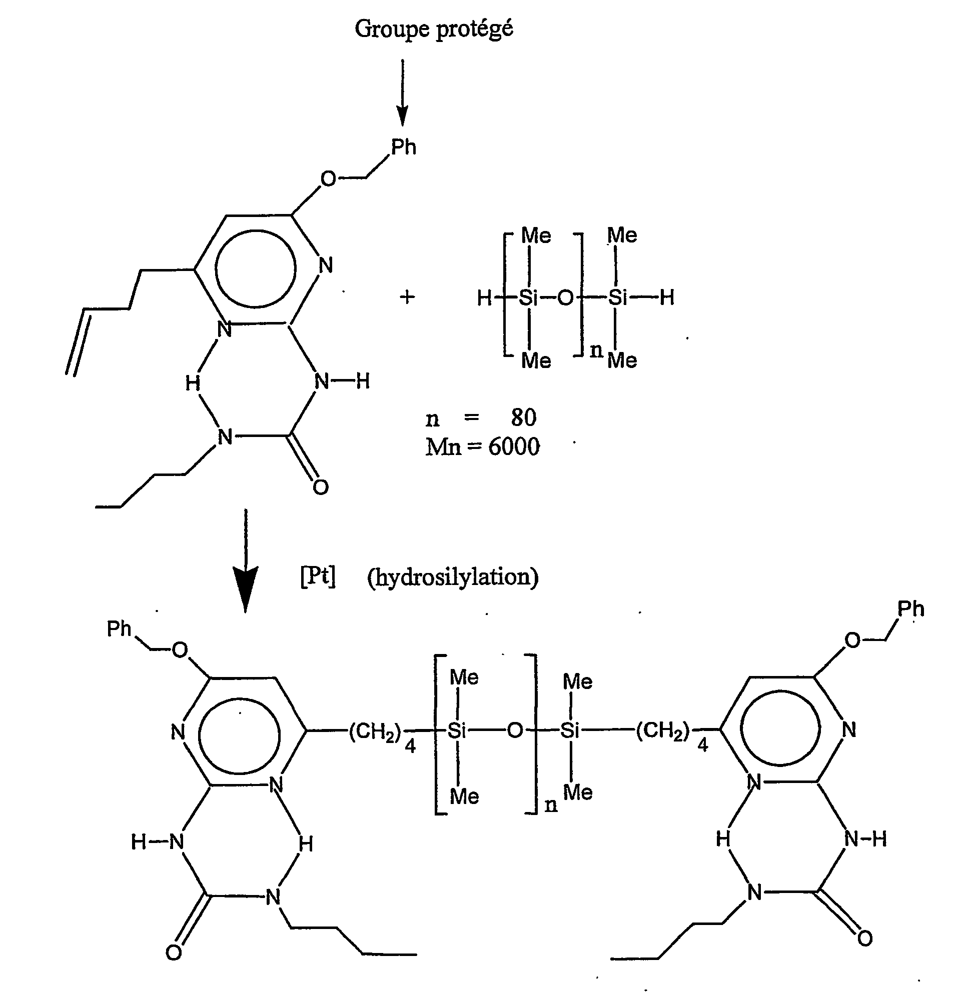

### Suivi de protection des groupes phényles par hydrogénation sur palladium

Ces réactions de protection, hydrosilylation puis déprotection sont détaillés dans l'article de J.H.K.K. HIRSCHBERG, Macromolécules, 32, 2696-2705 (1999) et dans la publication internationale WO-98/14504 de DSM.

### Formes galéniques

Les compositions utilisables dans l'invention peuvent se présenter sous toutes les formes galéniques classiquement utilisées pour une application topique et notamment sous forme d'une solution ou suspension aqueuse, alcoolique ou hydroalcoolique ou d'une solution huileuse ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle de type crème (H/E) ou (E/H), ou d'un gel aqueux ou anhydre, d'un onguent, ou de toute autre forme cosmétique.

Les compositions selon l'invention comportent généralement un milieu physiologiquement acceptable, c'est à dire compatible avec les tissus cutanés comme la peau et les matières kératiniques.

Le milieu physiologiquement acceptable est de avantageusement un milieu ne nuisant pas aux propriétés de persistance accrue d'au moins un effet cosmétique et/ou de soin, d'adhésion sur les matières kératiniques et de facilité de démaquillage apportées par la composition après application.

De préférence, le milieu physiologiquement acceptable est un milieu solubilisant des polymères selon 'l'invention comprenant au moins un solvant.

Parmi les solvants utilisables selon l'invention, on peut citer l'eau, les alcools et de préférence les alcools courts, les polyols, les esters courts, les huiles hydrocarbonées, les huiles de silicone, les huiles de silicone fluorées, et leurs mélanges. Les huiles peuvent être polaires ou apolaires.

Parmi ces solvants, on peut citer à titre d'exemple, les alcanols inférieurs en C₁-C₄, tels que l'éthanol et l'isopropanol, les polyols, les éthers de glycols, comme le 2 butoxyéthanol, l'éthylèneglycol, la glycérine, le propylèneglycol, le monoéthyléther et le monométhyléther de diéthylèneglycol, ainsi que les alcools aromatiques comme l'alcool benzylique ou le phénoxyéthanol et les produits analogues ou leurs mélanges.

Parmi les huiles polaires, on peut citer les huiles hydrocarbonées comportant des fonctions esters, éthers, acides, alcools ou leurs mélanges, telles que par exemple:
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat; ou encore les triglycérides des acides caprylique/caprique comme ceux vendus par la société Stearineries Dubois ou ceux vendus sous les dénominations Miglyol 810, 812 et 818 par la société Dynamit Nobel;
- les huiles de synthèse de formule R¹COOR² dans laquelle R¹ représente le reste d'un acide gras supérieur, linéaire ou ramifié, comportant de 7 à 19 atomes de carbone, et R² représente une chaîne hydrocarbonée ramifiée contenant de 3 à 20 atomes de carbone, comme par exemple l'huile de Purcellin (octanoate de cétostéaryle), l'isononanoate d'isononyle, les benzoates d'alkyle en C₁₂-C₁₅.
- les esters et les éthers de synthèse comme le myristate d'isopropyle, le palmitate d'éthyl-2-hexyle, les octanoates, les décanoates ou les ricinoléates d'alcools ou de polyalcools,
- les esters hydroxylés comme le lactate d'isostéaryle, le malate de di-isostéaryle, et les esters du pentaérythritol;
- les alcools gras en C₈ à C₂₆ comme l'alcool oléique; et
- leurs mélanges.

Parmi les huiles apolaires, on peut citer :
- les huiles de silicone volatiles ou non, linéaires ou cycliques, liquides à température ambiante, telles que les polydiméthylsiloxanes (PDMS) comportant des groupements alkyle, alcoxy ou phényle, pendants et/ou en bout de chaîne siliconée et ayant de 2 à 24 atomes de carbone; les silicones phénylées, comme les phényl triméthicones, les phényl diméthicones, les phényl triméthylsiloxy diphénylsiloxanes, les diphényl diméthicones, les diphényl méthyldiphényl trisiloxanes, les 2- phényléthyl triméthylsiloxysilicates,
- les hydrocarbures ou les hydrocarbures fluorés/ou fluorocarbures, linéaires ou ramifiés d'origine synthétique ou minérale, comme les huiles volatiles, telles que les huiles de paraffine (par exemple les isoparaffines), et les hydrocarbures aliphatiques (par exemple l'isododécane), ou non volatiles et leurs dérivés, la vaseline, les polydécènes, le polyisobutène hydrogéné tel que le parléam, le squalane, et leurs mélanges.

Les solvants sont présents de préférence dans des proportions allant de 1 à 90% en poids, et en particulier de 5 à 70 % en poids par rapport au poids total de la composition.

La solubilité des polymères selon l'invention sera contrôlée par le choix des squelettes polymèriques -POL- et des groupes de jonction (A).

Les polymères de l'invention peuvent dans certains cas déjà interagir entre eux physiquement (en établissant un réseau d'interactions H) dans certains solvants ou mélanges de solvants. Cela dépend de la nature et des proportions de solvants ou mélanges de solvants utilisés. Ceci peut provoquer une augmentation indésirable de la viscosité de la formule et gêner son application (par exemple lotion, aérosol, etc ...).

Pour s'affranchir de ce problème de viscosité, on peut opérer de deux façons:
- soit en solubilisant le polymère selon l'invention dans un solvant volatil pouvant établir des interactions H avec des groupes de jonction (A) des polymères, par exemple en utilisant des alcools courts, des polyols volatils, de l'eau,
- soit en utilisant un milieu solubilisant diphasique, comme par exemple une émulsion eau-dans-huile (E/H) ou huile-dans-eau (H/E). et un couple de polymères selon l'invention sélectifs dont les groupes de jonction et les squelettes polymériques sont de nature chimique différentes, chaque polymère étant dissous dans une phase différente de l'autre (l'un dans l'eau, l'autre dans l'huile).

Le couple de polymères A-POL₁-A et B-POL₂-B est alors choisi de manière que :
- chacun des groupes de jonction (A) n'établit pas d'interaction H avec lui-même mais seulement avec (B),
- chacun des groupes de jonction (B) n'établit pas d'interaction H avec lui-même mais seulement avec (A),
- les groupes de jonction (A) et (B) n'établissent des interactions H que lorsqu'ils sont mis en contact, et
- les squelettes polymériques -POL₁- et -POL₂- sont choisis de manière que les polymères A-POL₁-A et B-POL₂-B peuvent être chacun véhiculé dans une phase distincte de l'émulsion, de sorte qu'ils ne puissent pas réagir dans l'émulsion.

L'interaction entre les deux polymères ne se fera qu'à l'application, à condition toutefois que les milieux solubilisants soient des solvants volatils ou puissent pénétrer dans le support kératinique.

De façon connue, la composition de l'invention peut contenir également les adjuvants habituels dans les domaines cosmétique et dermatologique dans la mesure où l'adjuvant n'altère pas les propriétés recherchées pour la composition de l'invention, tels que les gélifiants hydrophiles ou lipophiles, les actifs de soin hydrophiles ou lipophiles, les conservateurs, les antioxydants, les parfums, les charges, les neutralisants, les polymères autres que ceux définis préalablement, les émulsionnants et les co-émulsionnants.

Les quantités de ces différents adjuvants sont celles classiquement utilisées dans les domaines considérés, et par exemple de 0,01 à 30% du poids total de la composition. Ces adjuvants, selon leur nature, peuvent être introduits dans la phase grasse, dans la phase aqueuse, dans les vésicules lipidiques et/ou dans les nanoparticules.

Les émulsionnants et les coémulsionnants éventuellement utilisés dans la composition sous forme d'émulsion sont choisis suivant le sens de l'émulsion (E/H ou H/E) parmi ceux classiquement utilisés dans le domaine considéré.

Ces émulsionnants et coémulsionnants sont de préférence présents, dans la composition, en une proportion allant de 0,3 à 30% en poids, et de préférence de 0,5 à 20% en poids par rapport au poids total de la composition.

Comme émulsionnants et coémulsionnants utilisables dans l'invention, on peut citer par exemple les esters d'acide gras et de polyol tels que le stéarate de PEG-100, le stéarate de PEG-50 et le stéarate de PEG-40 ; le tristéarate de sorbitane, les stéarates de sorbitane oxyéthylénés comprenant par exemple de 20 à 100 OE, et par exemple ceux disponibles sous les dénominations commerciales Tween® 20 ou Tween® 60, et leurs mélanges tels que le mélange de mono-stéarate de glycéryle et de stéarate de polyéthylène glycol (100 OE) commercialisé sous la dénomination SIMULSOL 165 par la société SEPPIC.

On peut citer aussi les émulsionnants siliconés tels que les diméthicone copolyols et les alkyl diméthicone copolyols. On peut citer par exemple comme diméthicone copolyol, le mélange de diméthicone copolyol, de cyclométhicone et d'eau (10/88/2), commercialisé par la société Dow Corning sous la dénomination DC3225C ou DC2-5225C, et comme alkyl diméthicone copolyol, ceux ayant un radical alkyle comportant de 10 à 22 atomes de carbone, tels que le cétyl diméthicone copolyol comme le produit commercialisé sous la dénomination Abil EM-90 par la société Goldschmidt et le mélange de diméthicone copolyol et de cyclopentasiloxane (85/15) commercialisé sous la dénomination Abil EM-97 par la société Goldschmidt ; le lauryl diméthicone copolyol et par exemple le mélange d'environ 91% de lauryl diméthicone copolyol et d'environ 9% d'alcool isostéarylique, commercialisé sous la dénomination Q2-5200 par la société Dow Corning, et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles.

Comme gélifiants lipophiles, on peut citer les argiles modifiées comme les bentones, les sels métalliques d'acides gras et la silice hydrophobe.

Comme actifs de soin, on peut utiliser notamment les dépigmentants, les émollients, les hydratants, les anti-séborrhéiques, les anti-acnéiques, les agents favorisant la repousse des cheveux, les agents kératolytiques et/ou desquamants, les agents antirides et tenseurs, les agents anti-irritants, les agents apaisants, les vitamines, les filtres, les absorbeurs d'odeur et leurs mélanges.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

La composition selon l'invention peut se présenter sous la forme d'une composition de soin, notamment hydratant, pour matières kératiniques comme la peau, les lèvres et/ou les phanères, et/ou sous forme d'une composition d'hygiène corporelle notamment sous forme de produit déodorant ou démaquillant, ou sous forme d'un produit de maquillage des matières kératiniques, et/ou sous forme d'un produit de nettoyage, et/ou sous forme d'un produit capillaire, par exemple un shampooing ou un après-shampooing ou encore un produit de coiffage.

Avantageusement, la composition contient au moins une matière colorante. Cette matière colorante peut représenter de 0,01 à 50% en poids, de préférence de 0,5 à 40% en poids par rapport au poids total de la composition.

Comme matières colorantes utilisables selon l'invention, on peut citer les colorants lipophiles, les colorants hydrophiles, les pigments et les nacres habituellement utilisés dans les compositions cosmétiques ou dermatologiques, et leurs mélanges.

Les colorants liposolubles sont, par exemple, le rouge Soudan, le DC Red 17, l DC Green 6, le carotène, l'huile de soja, le brun Soudan, le DC Yellow 11, le DC Violet 2, le DC orange 5, le jaune quinoléine et le rocou. Ils peuvent représenter de 0,01 à 10% du poids de la composition, et mieux de 0,05 à 5%.

Les pigments peuvent être blancs ou colorés, minéraux et/ou organiques, enrobés ou non.

On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique.

Les pigments minéraux préférés sont les oxydes de fer, notamment l'oxyde de fer rouge, l'oxyde de fer jaune, l'oxyde de fer rouge et jaune, l'oxyde de fer brun, l'oxyde de fer noir et le dioxyde de titane.

Parmi les pigments organiques, on peut citer:
- le noir de carbone,
- les pigments de type D&C, tel que le D&C Red No 36, et
- les laques à base de carmin de cochenille, de baryum, de strontium, de calcium telle que le D&C Red No 7 calcium lake, d'aluminium, telles que le D&C Red No 27 aluminium lake, le D&C Red No 21 aluminium lake, le FD&C Yellow No 5 aluminium lake, le FD&C Yellow No 6 aluminium lake, le D&C Red No 7 et le FD&D Blue No 1.

Les pigments peuvent représenter de 0,01 à 40% du poids total de la composition, et de préférence de 1 à 30%.

Les pigments nacrés peuvent être choisis parmi les pigments nacrés blancs, tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité, ainsi que les pigments nacrés à base d'oxychlorure de bismuth. Ils peuvent représenter de 0,01 à 40% du poids total de la composition et mieux de 0,1 à 30%.

La composition de l'invention peut se présenter sous la forme d'un produit de maquillage, en particulier coloré, de la peau, en particulier un fond de teint, présentant éventuellement des propriétés de soin, un blush, un fard à joues ou à paupières, un produit anti-cerne, un eye-liner ; un produit de maquillage des lèvres comme un rouge à lèvres, présentant éventuellement des propriétés de soin, un brillant à lèvres, les crayons à lèvres ; un produit de maquillage des phanères comme les ongles, les cils en particulier sous forme d'un mascara pain, les sourcils et les cheveux sous forme de crayon ; un produit de tatouage temporaire de la peau du corps.

La composition se présente de préférence sous forme d'un produit coloré pour les lèvres.

La composition de l'invention peut également se présenter sous la forme d'un produit de coloration capillaire.

Elle peut aussi se présenter sous forme d'un produit de soin, non coloré, destiné à traiter la peau et notamment à l'hydrater, la lisser, la dépigmenter, la nourrir, la protéger des rayons solaires, ou lui conférer un traitement spécifique. A cet effet, elle contient avantageusement au moins un actif de soin choisi parmi les dépigmentants, les émollients, les hydratants, les anti-séborrhéiques, les anti-acnéiques, les agents favorisant la repousse des cheveux, les agents kératolytiques et/ou desquamants, les agents antirides et tenseurs, les agents anti-irritants, les agents apaisants, les vitamines, les filtres, les absorbeurs d'odeur et leurs mélanges.

L'invention a également pour objet un procédé pour améliorer à la fois la persistance d'au moins un effet apporté après dépôt par une composition cosmétique et l'adhésion de la composition appliquée sur les matières kératiniques, et pour permettre également une élimination rapide, totale et sélective du dépôt, qui consiste à ajouter à la composition une quantité efficace d'au moins un polymère linéaire, ramifié ou cyclique, ou dendrimère, comportant :
- un squelette polymérique -POL- comprenant au moins deux motifs de répétition, et
- au moins deux groupes de jonction (A), identiques ou différents, latéraux, dans la chaîne, ou terminaux, fixés sur le squelette polymérique et capables d'établir au moins trois liaisons H avec un ou plusieurs groupes de jonction partenaires, de nature chimique identique ou différente,
chaque appariement de deux groupes de fonction (A) faisant intervenir au moins trois liaisons H.

L'élimination du dépôt peut consister notamment en le rinçage d'une composition nettoyante ou en un démaquillage d'un dépôt de maquillage (rouge à lèvres, fond de teint, mascara, eye-liner notamment).

L'invention a aussi pour objet l'utilisation telle que définie selon la revendication 22.

L'invention est illustrée plus en détail dans les exemples suivants. Les quantités sont données en pourcentage massique.

### EXEMPLES

### EXEMPLE 1

### Réalisation d'un copolymère POE/PPO bifonctionnel possédant trois extrémités uréïdopyrimidones

La synthèse du copolymère POE/PPO trifonctionnel est en tout point identique à celle décrite dans l'article de R.F.M. LANGE, J. Polym. Sci.: Part A= Polym. Chem., 37, 3657-70 (1999).

### Réactifs

- **Composé 1** :
   copolymère POE/PPO à trois extrémités OH, de structure répondant à la formule (XXXII) : 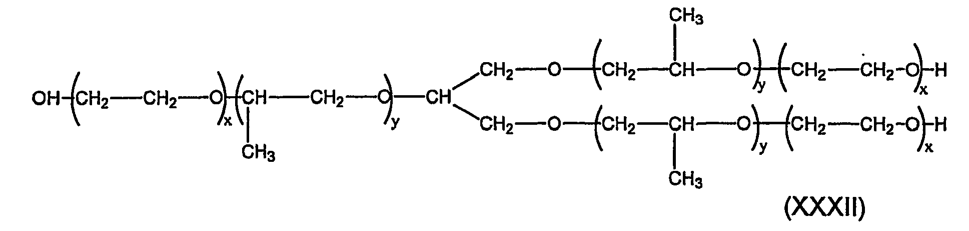 où x et y sont tels que le poids moléculaire moyen en nombre est de 600, commercialisé par la société ARCO.
- **Composé 2 :**
   isophérone diisocyanate commercialisé par la société BAYER, de structure : 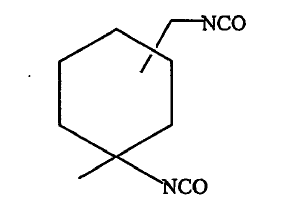
- **Composé 3** :
   méthyl isocytosine 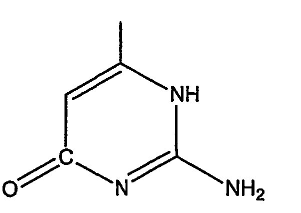 commercialisé par la société FLUKA.
- **Solvant :** CHCl₃
- Pyridine

### Mode opératoire

On ajoute une quantité catalytique (environ 30 mg) de Ti(OnBu)_{y} à une solution contenant 25,4 g (soit 5,3 mmol) du composé 1 dans 50 ml de CHCl₃ et on agite le mélange pendant 10 minutes. Puis on ajoute goutte à goutte 3,24g (soit 16,7 mmol) du composé 2 et on agite le mélange pendant 14 heures à la température ambiante. Des analyses infrarouge (lR) du mélange réactionnel ont révélé qu'il ne restait plus de fonctions hydroxyles du composé 1 libre. On élimine le solvant par évaporation sous pression réduite.

On ajoute ensuite au mélange réactionnel 50 ml de pyridine et 2,25g (soit 18,0 mmol) du composé 3 et on laisse réagir le mélange réactionnel au reflux pendant 18 heures. Des analyses IR montrent qu'il n'y a plus de groupes isocyanates libres. On élimine le solvant (pyridine) par évaporation sous vide. Le produit de réaction brut est dissous dans le CHCl₃, filtré, puis précipité dans du n-hexane, et enfin purifié par filtration au travers une fine couche de silice : on obtient alors 28,5g de copolymère POE/PPO à trois extrémités uréïdopyrimidone avec un rendement réactionnel de 95%.

### EXEMPLE 2

### Réalisation d'un polydiméthylsiloxane (n=80) ayant deux extrémités uréïdopyrimidone

La synthèse de ce composé est en tout point identique à celles rapportée dans l'article de J.H.K.K. HIRSCHBERG, Macromolecular, 32, 2696-2705, (1999), et dans la demande internationale WO-98-14504 (exemples 9, 10 et 13, aux pages 18, 19, et 22 respectivement).

On rapportera ici la synthèse citée dans l'article de HIRSCHBERG, réalisée avec un polyhydrogénoorganosiloxane (n=80) pour donner le produit final 1 : n = 100. La synthèse du polydiméthyl siloxane ayant deux extrémités uréïdopyrimidone se décompose en quatre étapes étapes:
- la première étape consistant en la préparation du 6-(3-butenyl)isocytosine (composé 4), illustrée par le schéma 1,
- la deuxième étape consistant en la préparation du 4-benzyloxy-6-(3-butenyl)-2-butylgluréidopyrimidone (composé 6), illustrée par le schéma 2,
- la troisième étape consistant en la préparation du α,ω-Di(4-benzyloxy-6-butyl-2-butylureidopyrimidinyl)poly(dimethylsiloxane) (composé 2-Bn), illustrée par le schéma 3, et
- la quatrième étape consistant en la préparation du polydiméthylsiloxane (composé 2), illustrée par le schéma 4.

- **Composé 2 :**
   α,ω-Di(6-butyl-2-butylureido-4-pyrimidinoyl)poly(diméthylsiloxane)
- **Composé 2-Bn :**
   α,ω-Di(4-benzyloxy-6-butyl-2-butylureidopyrimidinyl)poly-(dimethylsiloxane)
- **Composé 3 :**
   Ethyl 3-oxo-6-heptenoate
- **Composé 4 :**
   6-(3-butenyl)isocytosine
- **Composé 5 :**
   6-(3-butenyl)-2-butylureido-4-pyrimidinone.
- **Composé 6 :**
   4-Benzyloxy-6-(3-butenyl)-2-butylureidopyrimidine

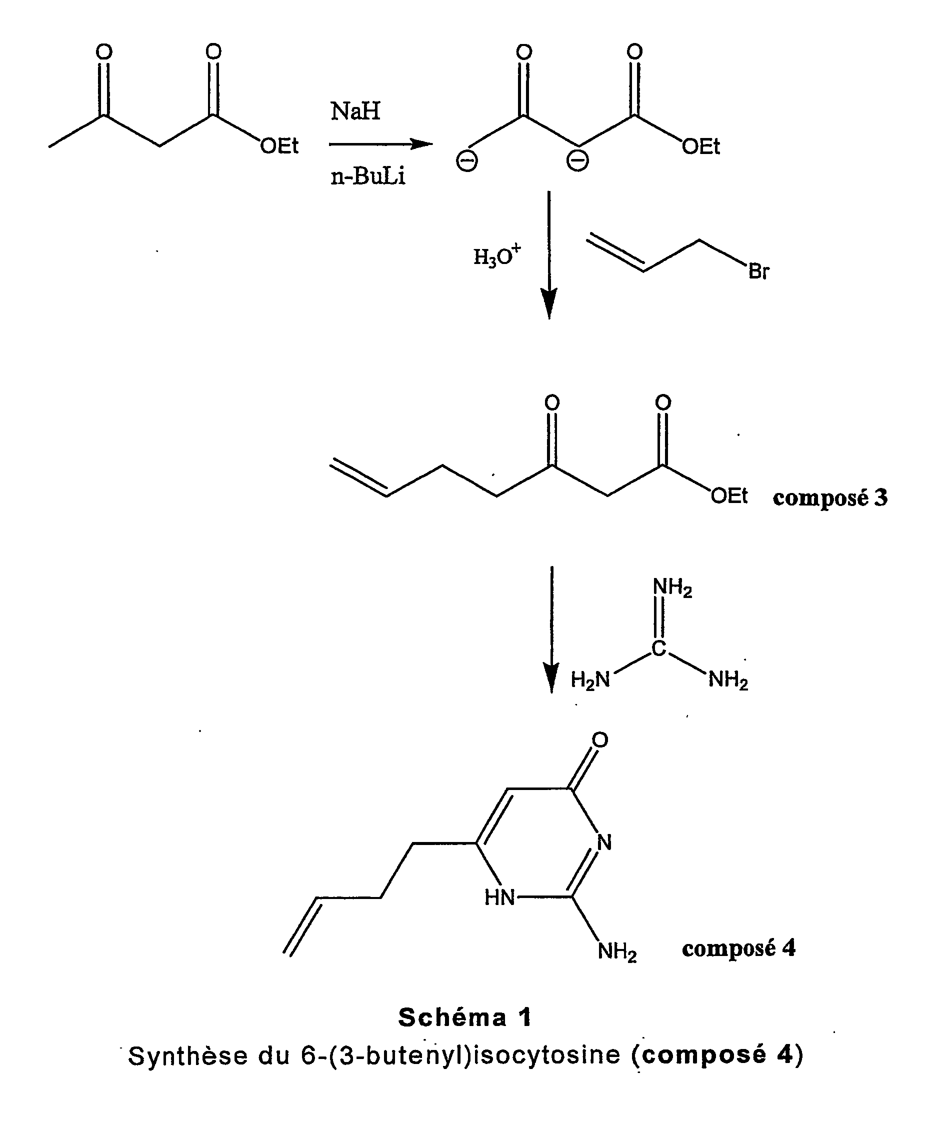

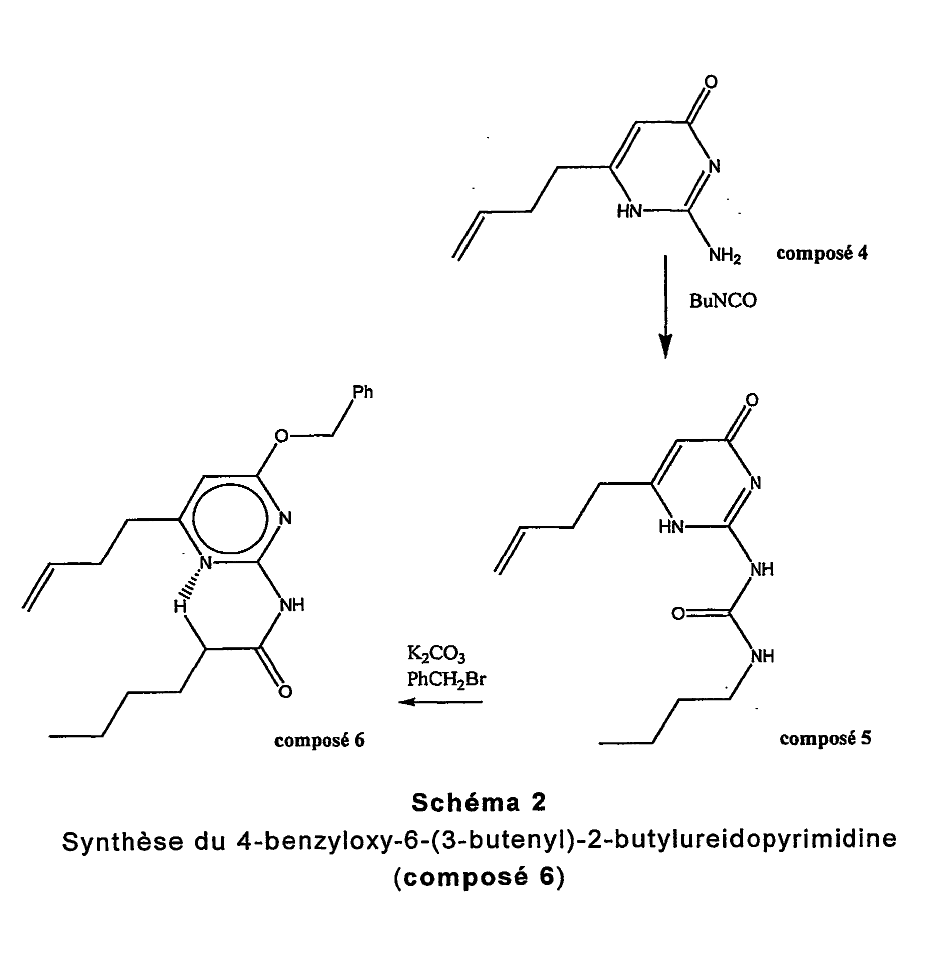

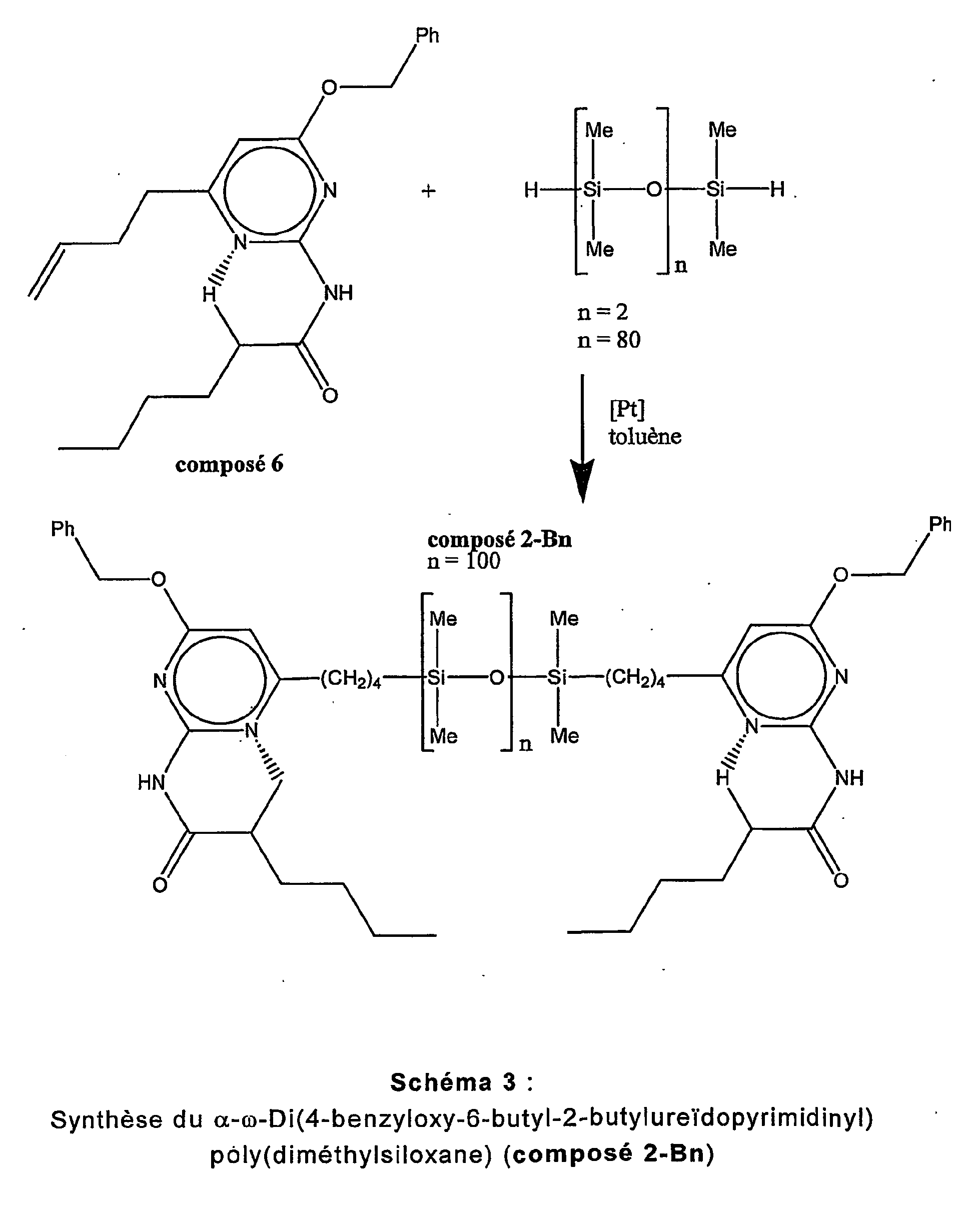

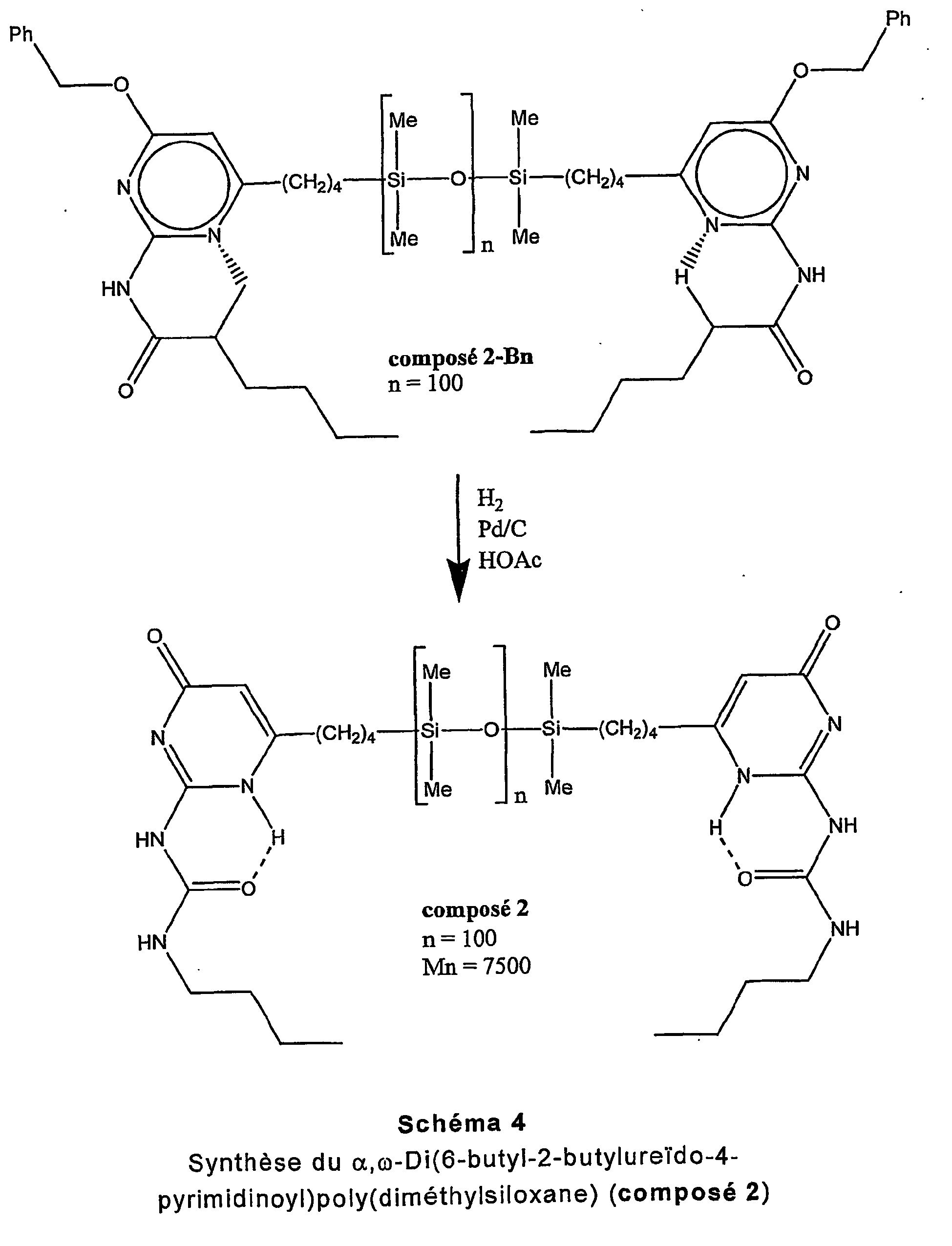

### Mode opératoire

### Obtention du composé 3

Le composé 3 est préparé conformément à la méthode décrite dans l'article de HUCHIN, S.N., J. Am. Chem. Soc., 96 1082, 1974 (bp 106-108°C, 15 mmHg, lit. 106-108°C). ; ¹H NMR (CDCl₃): δ 5.82 (m, 1 H, HC=CH₂), 5.01 (m, 2H, CH2=CH), 4.20 (q, 2H, OCH₂), 3.45 (s, 2H, CH₂), 2.66 (t, 2H, CH₂), 2,34 (m, 2H, CH₂), 1.30 (t, 3H, CH₃) ppm. ; ¹³C NMR (CDCl₃) : δ 202.4, 167.6, 137.1, 116.04, 61.9, 49.9, 42.5, 27.92, 14,6 ppm. ; IR (Kbr); ν=3040, 2981, 1744, 1641, 1236 cm⁻¹.

### Obtention du composé 4

Dans un réacteur à trois cols on introduit 18,02g de carbonate de guanidine (soit 0,10 mol). Balayage par un courant d'azote, puis obturation par un septum. On ajoute 200 ml d'éthanol sec. Puis, sous agitation, on introduit lentement l'éthyl-3-oxo-6-heptenoate (provenant de la réaction de 26g, 0,2 mol d'éthyle acétoacétate). Le mélange est ensuite agité vigoureusement, puis chauffé au reflux pendant 24 heures. Une partie du solvant est distillée et le produit est précipité par addition d'eau. Le précipité est filtré, lavé à l'eau, à l'éthanol froid et l'acétone froid et séché sous vide (13,73g, 42%).

### Obtention du composé 5

On ajoute lentement 2,86 ml de butyl isocyanate (soit 25,4 mmol) à une solution contenant 3,0g de composé 4 (soit 18,2 mmol) dans 36 ml de pyridine (séchée). Le mélange est chauffé au reflux pendant 3 heures. La pyridine est éliminée par évaporation. Le produit brut est recristallisé à partir de l'éthanol pour donner 4,2g (87%) de composé 5.

### Obtention du composé 6

On ajoute, en utilisant pour cela une seringue, 1,58 ml de bromure de benzyle (soit 13,24 mmol) à une suspension contenant 1,0g du composé 5 (soit 3,8 mmol) et 1,83g de carbonate de potassium (soit 13,24 mmol) dans 25 ml de DMF (sec). La suspension est agitée vigoureusement à une température de 80°C pendant 24 heures. On ajoute ensuite de l'acétone dans le mélange réactionnel et on filtre la suspension ainsi obtenue. Le résidu est lavé avec le DMF et l'acétone. Puis, on ajoute de l'eau au filtrat. Le précipité blanc est filtré et lavé à l'eau. La recristallisation à partir du mélange méthanol/eau (3 : 1 v/v), suivie de la recristallisation à partir de l'hexane donne 1,04g de composé 6 pur (soit un rendement réactionnel de 78%).

### Obtention du composé 2-Bn

Dans une boîte à gants, on réalise une solution par mélange de :
- 27g de poly(diméthylsiloxane)dihydrure, soit 4,5 mmole (provenance ABCR, masse moléculaire moyenne en nombre de 6000),
- 23,92g, soit 67.49 mmole, du composé 6,
- 136 µl d'une solution de catalyseur au platine PCO72 (provenance ABCR) à 2,1-2,4% dans le xylène, et
- 135 ml de toluène.

Sortie de la boîte à gants, on chauffe le récipient contenant cette solution à 80°C pendant 24 heures sous atmosphère d'azote.

Le produit obtenu est évaporé de son solvant, puis lavé trois fois par 30 ml de méthanol. On obtient alors 24.42g du composé 2-Bn sous forme d'une huile de faible viscosité ne présentant pas d'impureté à l'analyse RMN (soit un rendement de 90%).

### Obtention du composé 2

On mélange ensemble 10g de composé 2-Bn (soit 1,17 mmol), 0,11g de Pd/C (10% en poids, 0,1 mmol), 100 ml de THF, 50 ml d'éthanol et 0,03g d'acide acétique (soit 0,5 mmol), chargés dans un réacteur Parr. Le mélange réactionnel est lavé plusieurs fois à l'azote, puis à l'hydrogène. Le réacteur a été agité pendant 24 heures sous atmosphère hydrogène à une pression d'environ 4 atm. Après filtration du catalyseur, le solvant est évaporé. Le produit brut de réaction est alors dissous dans un mélange dichlorométhane/éthanol (3 :1), et le dichlorométhane est ensuite évaporé. Le composé final précipite au fond du flacon et la couche supérieure constituée d'éthanol, est éliminée. Après séchage sous vide, on obtient 9g d'huile très visqueuse. Contrôle de la pureté par RMN.

### EXEMPLE 3

### Préparation d'un polyester présentant deux extrémités α,ω à groupes uréïdopyrimidone

### A) Synthèse du polyester à extrémités initiales α,ω-OH: copolymère sébaçate/téréphtalate de néopentylglycol (PM=600-700) et à extrémités α,ω -OH

### réactifs

- Diméthyl téréphtalate 116,4 g (0,6 M)
- Diméthyl sébaçate 138g (0,6 M)
- 2,2-diméthyl-1,3-propanediol
   (ou néopentylglycol) 274,6 g (2,64 M)
- Acétate de zinc dihydraté 1,6 g
(0,3% en poids/réactif)
- 1,dichloroéthane pur 2 λ
- Eau permutée 2 λ

### Mode opératoire

On utilise un réacteur de 500 ml cylindrique muni d'une arrivée d'azote, d'un thermomètre et d'un montage de distillation. Le réacteur est chauffé par un bain en alliage de Wood.

On introduit dans le réacteur le 2,2-diméthyl-1,3-propanediol (point de fusion 126-128°C) et le diméthylsébaçate (point de fusion 25-28°C) préalablement fondu. On chauffe le mélange de la température ambiante à 150°C en une heure. Dès que le milieu réactionnel devient limpide vers 100°C, on ajoute le diméthyl téréphtalate. Dès que l'on atteint 150°C dans la réaction, on introduit alors l'acétate de zinc. On laisse se développer la synthèse pendant trois heures à 150°C tout en recueillant le méthanol formé. Ensuite, on monte la température à 200°C en quarante-cinq minutes et on maintient alors trois heures à 200°C. La distillation du méthanol continue durant toute la condensation.

A la fin des trois heures à 200°C, on laisse revenir à la température ambiante en réduisant l'agitation. Dès que la température intérieure arrive à 50°C, on ajoute 300 ml de 1,2-dichloroéthane.

On récupère la solution de synthèse que l'on dilue par 1,7 l de 1,2-dichloroéthane. L'excès de 2,2-diméthyl-1,3-propanediol est alors extrait deux fois par un litre d'eau permutée. Durant ces deux extractions, il est possible qu'il se forme une émulsion interfaciale: Cette émulsion est facilement éliminable par chauffage de l'eau, utilisée en cours de l'extraction. On récupère la phase organique que l'on sèche sur sulfate de sodium anhydre. On filtre et on évapore le 1,2-dichloroéthane jusqu'à la récupération du produit sec.

Le polymère se présente sous forme de pâte à température ambiante et devient liquide à 50°C.

### Caractérisation :

| | |
|---|---|
| Indice d'hydroxyle : | 189-192 |
| Poids moléculaire mesuré en sommet de pic par chromatographie par exclusion stérique : | 600 |

### B) Transformation des extrémités α,ω-OH- en extrémités α,ω uréidopyrimidone

On opère exactement comme pour l'exemple N°1 par réaction successive avec l'isophorone diisocyanate, puis introduction de méthylisocytosine, en adaptant les quantités au taux d'extrémité -OH contenues dans l'oligomère.

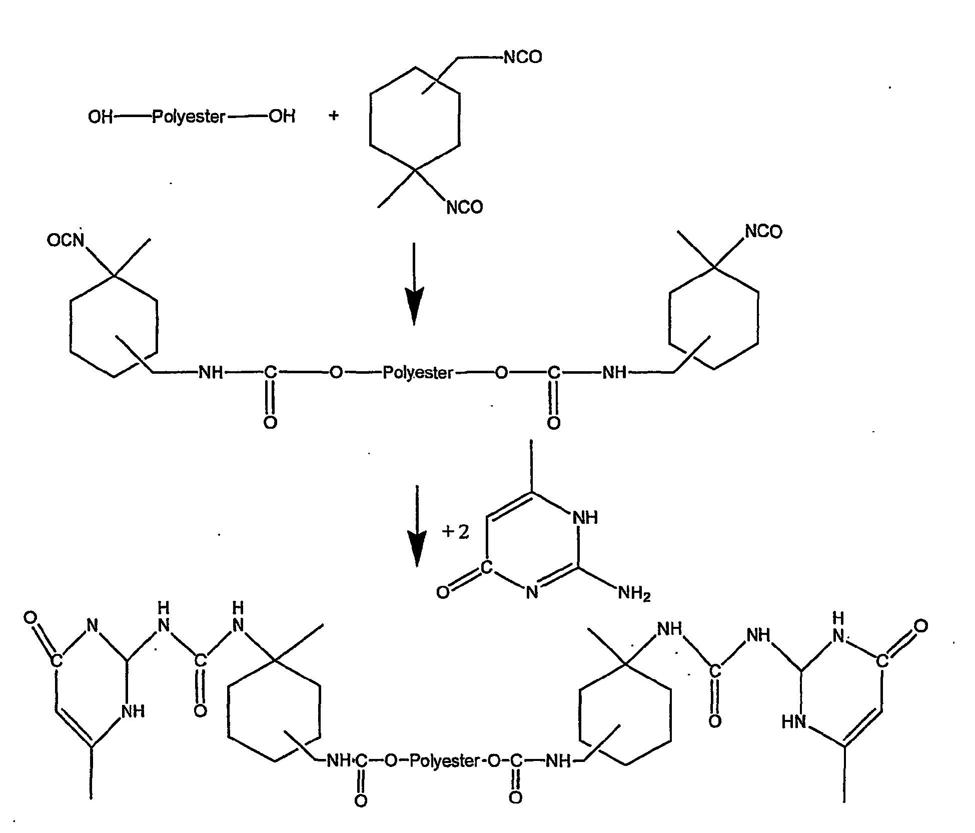

### EXEMPLES DE COMPOSITION COSMÉTIQUE

### EXEMPLE A

### Rouge à lèvres enstich

On prépare un rouge à lèvres ayant la composition suivante :

| | |
|---|---|
| Cire de polyéthylène PERFORMALENE 500^{®(1)} | 15 g |
| PDMS à extrémités uréïdopyrimidone de l'exemple 2 | 5 g |
| Pigments | 9 |
| Huile de Polyisobutylène hydrogéné⁽²⁾ | 35,5 g |
| Huile de phényltriméthicone Dow 556 Fluid^{®(3)} | 35,5 g |

| | |
|---|---|
| ⁽¹⁾ vendue par la société PETROLITE ⁽²⁾ de viscosité 34 mm²/s (34 cSt) à 25°C, vendue sous le nom de « Parléam^{®}» par la société NIPPON OIL-FATS. (3) vendue par la société DOW CORNING. | |

On mélange à 110°C tous les constituants de la composition ci-dessus dans un poêlon. Après homogénéisation et broyage des pigments, on coule le mélange dans un moule adéquat. On obtient ainsi un stick présentant de bonnes caractéristiques rhéologiques. Il permet de déposer sur les lèvres un film présentant une bonne tenue dans le temps (non virage de la couleur et non départ du film au contact de la salive et du frottement des lèvres).

### EXEMPLE B

### Brillant à lèvres

On prépare un brillant à lèvres ayant la composition suivante:

| | |
|---|---|
| PDMS à extrémités uréïdopyrimidone de l'exemple 2 | 5 g |
| Pigments (DC Red N°7 Calcium (laque)) | 5 g |
| Huile de phényltriméthicone Dow 556 Fluid^{®(*)} | 90 g |

| | |
|---|---|
| (*) commercialisé par la société DOW CORNING. | |

Le PDMS de l'exemple 2 est tout d'abord dissous dans l'huile contenue dans un récipient. On obtient un brillant à lèvres en dispersant les pigments dans cette phase huileuse. Le brillant à lèvres ainsi obtenu peut être appliqué au pinceau sur les lèvres. Il confère aux lèvres une coloration brillante, durable dans le temps.

### EXEMPLE C

### Gel de coiffage

On prépare un gel de coiffage ayant la composition suivante :

| | |
|---|---|
| Copolymère POE/PPO uréïdopyrimidone de l'exemple 1 | 0,2 g |
| Polyacrylate réticulé Synthalen K^{(*)} | 1 g |
| neutralisé avec Aminométhylpropanol éthanol | 17 g |
| eau | qsp 100g |

| | |
|---|---|
| (*) commercialisé par la société 3V | |

## Revendications

1. Composition cosmétique de soin, et/ou de traitement, et/ou de maquillage des matières kératiniques, comprenant, dans un milieu physiologiquement acceptable, une quantité efficace d'au moins un polymère linéaire, ramifié ou cyclique, ou dendrimère, comportant :
• un squelette polymérique -POL- comprenant au moins deux motifs de répétition, et
• au moins deux groupes de jonction (A) fixés sur le squelette polymérique et capables d'établir des liaisons H avec un ou plusieurs groupes de jonction partenaires, de nature chimique identique ou différente, chaque appariement de deux groupes de jonction faisant intervenir au moins trois liaisons H,
**caractérisée en ce que** les groupes de jonction (A) sont choisis parmi : adénine, guanine, cytidine, thymine, ptérine, uréidopyrimidone, mélamine, acide cyanurique, maléïmide, phtalhydrazide, isoguanine, glycoluril, uracil, acylaminopyridine, de préférence monoacylamino pyridine et Bis(acylamino) pyridine, acylaminotriazine, de préférence mono-acyl 2,4-diamino-s-triazine et Bis-acyl 2,4-diamino-s-triazine, pyridine/phénol, urazole, glutarimide, acide urazoyl benzoïque, succinimide, 2,4-diamino-s-triazine, uréïdotriazine, phtalhydrazide, acide urazoyl benzoïque.

2. Composition selon la revendication 1, **caractérisée en ce que** le polymère est :
• linéaire ou ramifié et fonctionnalisé uniquement aux extrémités, ou
• linéaire ou ramifié et comportant au moins deux groupes de jonction (A) dans la chaîne, ou
• linéaire et portant les groupes de jonction (A) en ramifications latérales, ou
• dendrimère, ou en étoile, et portant les groupes de jonction (A) aux extrémités.

3. Composition selon la revendication 2, **caractérisée en ce que** le polymère est tri ou multifonctionnel, et de préférence en étoile ou hyperbranché ou ramifié, de sorte que la composition se présente sous forme de film après application sur les matières kératiniques.

4. Composition selon l'une quelconque des revendications 1 à 3, **caractérisée en ce que** le polymère comporte au moins un groupe de jonction (A₁) et au moins un groupe de jonction complémentaire (A₂), chaque groupe de jonction (A₁) pouvant établir au moins trois liaisons H avec chaque groupe complémentaire (A₂), les groupes de jonction (A₁) et (A₂) étant choisis par couple parmi les couples :
• Bis(acylamino)pyridine/uracil ou succinimide, ou thymine, ou glutarimide, ou acide cyanurique, ou leurs mélanges,
• 2,4-diamino-s-triazine/ uracil ou succinimide, ou thymine, ou glutarimide, ou acide cyanurique, ou leurs mélanges,
• monoacyl-2,4-diamino-s-triazine/ uracil ou succinimide, ou thymine, ou glutarimide, ou acide cyanurique, ou leurs mélanges,
• mélamine/ uracil ou succinimide, ou thymine, ou glutarimide, ou acide cyanurique, ou leurs mélanges.

5. Composition selon l'une quelconque des revendications précédentes, **caractérisée en ce que** le squelette polymérique -POL-a un degré de polymérisation allant de 2 à 70000, et de préférence de 10 à 5000.

6. Composition selon l'une quelconque des revendications précédentes, **caractérisée en ce que** le squelette polymérique -POL-est choisi parmi les polydiènes notamment hydrogénés, les polyesters, les polycarbonates, les polyacétals, les polyoxyalkylènes, les polythioéthers, les perfluoropolyéthers, les polyoléfines, les polyorganosiloxanes, les polymères vinyliques, les poly(meth)acryliques, les dérivés cellulosiques, les dérivés polysaccharides, les hyperbranchés ou dendrimères.

7. Composition selon l'une quelconque des revendications précédentes, **caractérisée en ce que** le polymère est un copolymère POE/PPO bifonctionnel présentant trois extrémités uréïdo-pyrimidone.

8. Composition selon l'une quelconque des revendications 1 à 6, **caractérisée en ce que** le polymère est un polydiméthylsiloxane présentant deux extrémités uréïdopyrimidone.

9. Composition selon l'une quelconque des revendications 1 à 6, **caractérisée en ce que** le polymère est un polyester présentant deux extrémités α,ω-uréïdopyrimidone.

10. Composition selon l'une quelconque des revendications précédentes, **caractérisée en ce que** la composition se présente sous forme d'une solution ou suspension aqueuse, alcoolique ou hydroalcoolique, ou d'une solution ou suspension huileuse, ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle de type crème (H/E) ou (E/H), ou d'un gel aqueux ou anhydre, d'un onguent, d'une poudre libre ou compactée à utiliser telle quelle ou à incorporer dans un excipient.

11. Composition cosmétique selon l'une quelconque des revendications précédentes, **caractérisée en ce qu'**elle contient au moins un solvant choisi parmi l'eau, les alcools, les polyols, les esters courts, les huiles hydrocarbonées, les huiles de silicone, les huiles de silicone fluorées, et leurs mélanges.

12. Composition selon l'une quelconque des revendications précédentes, **caractérisée en ce qu'**elle comprend au moins un adjuvant choisi parmi les gélifiants hydrophiles ou lipophiles, les actifs hydrophiles ou lipophiles, les conservateurs, les antioxydants, les solvants, les parfums, les charges, les filtres hydrophiles, les absorbeurs d'odeur, les neutralisants, les polymères autres que ceux définis selon l'une quelconque des revendications précédentes, et les émulsionnants.

13. Composition selon la revendication 12, **caractérisée en ce que** les actifs sont choisis parmi les dépigmentants, les émollients, les hydratants, les anti-séborrhéiques, les anti-acnéiques, les agents favorisant la repousse des cheveux, les agents kératolytiques et/ou desquamants, les agents antirides et tenseurs, les agents anti-irritants, les agents apaisants, les vitamines, les filtres, les absorbeurs d'odeur et leurs mélanges.

14. Composition selon l'une quelconque des revendications précédentes, **caractérisée en ce qu'**elle constitue une composition de soin, pour matières kératiniques comme la peau, les lèvres et/ou les phanères, et/ou sous forme d'une composition d'hygiène corporelle tel qu'un produit déodorant ou un démaquillant, ou sous forme d'un produit de maquillage des matières kératiniques, et/ou sous forme d'un produit de nettoyage, et/ou sous forme d'un produit capillaire.

15. Composition selon l'une quelconque des revendications précédentes, **caractérisée en ce qu'**elle contient au moins une matière colorante choisie parmi les colorants lipophiles, les colorants hydrophiles, les pigments, les nacres et leurs mélanges.

16. Composition selon l'une quelconque des revendications précédentes, **caractérisée en ce qu'**elle se présente sous forme de fond de teint, de blush, de fard à joues ou à paupières, de produit anti-cerne, d'eye-liner, de produit de tatouage temporaire de la peau du corps, de produit de maquillage des lèvres comme un rouge à lèvres, de brillant à lèvres, de crayon à lèvres, ou sous forme de produit de maquillage des phanères, ou sous forme d'un produit de coloration capillaire.

17. Procédé cosmétique de soin, de maquillage ou de traitement des matières kératiniques des êtres humains, comprenant l'application sur les matières kératiniques d'une composition cosmétique, telle que définie selon l'une quelconque des revendications précédentes.

18. Procédé pour améliorer à la fois la persistance d'au moins un effet apporté après dépôt par une composition cosmétique et l'adhésion de la composition appliquée sur les matières kératiniques, et pour permettre également un démaquillage rapide, total et sélectif, qui consiste à ajouter à la composition une quantité efficace d'au moins un polymère linéaire, ou ramifié, ou cyclique, ou dendrimère ou hyperbranché, comportant :
• un squelette polymérique (POL) comprenant au moins deux motifs de répétition, et
• au moins deux groupes de jonction (A) fixés sur le squelette polymérique et capables d'établir au moins trois liaisons H avec un ou plusieurs groupes de jonction partenaires, de nature chimique identique ou différente, chaque appariement de deux groupes de jonction (A) faisant intervenir au moins trois liaisons H.

19. Utilisation d'une quantité efficace d'au moins un polymère linéaire, ramifié ou cyclique, ou dendrimère, comportant :
• un squelette polymérique -POL- comprenant au moins deux motifs de répétition, et
• au moins deux groupes de jonction (A) fixés sur le squelette polymérique et capables d'établir des liaisons H avec un ou plusieurs groupes de jonction partenaires, de nature chimique identique ou différente, chaque appariement de deux groupes de jonction faisant intervenir au moins trois liaisons H,
dans une composition cosmétique de soin et/ou de maquillage de la peau, des lèvres ou des phanères, comme agent pour améliorer la persistance d'un effet apporté après dépôt de la composition sur la peau, les lèvres ou les phanères, et/ou pour éliminer rapidement et sélectivement le dépôt de la composition.

## Claims

1. A care, and/or treatment, and/or make-up composition for keratin materials, comprising, in a physiologically acceptable medium, an efficient amount of at least one linear, branched or cyclic polymer, or dendrimer, comprising:
• a -POL- polymeric backbone comprising at least two repeating units, and
• at least two binding groups (A), fixed on the polymeric backbone and liable to provide H bonds with one ore more linking binding groups, of an identical or different chemical nature,each coupling of two binding groups involving at least three H bonds,
**characterized in that** the binding groups (A) are selected amongst adenine, guanine, cytidine, thymine, pterine, ureidopyrimidone, melamine, cyanuric acid, maleimide, phthalhydrazide, isoguanine, glycoluril, uracil, acylaminopyridine, preferably monoacylaminopyridine and Bis(acylamino) pyridine, acylaminotriazine, preferably mono-acyl 2,4-diamino-s-triazine and Bis-acyl 2,4-diamino-s-triazine, pyridine/phenol, urazole, glutarimide, urazoylbenzoic acid, succinimide, 2,4-diamino-s-triazine, ureïdotriazine, phtalhydrazide, urazoyl benzoïc acid.

2. A composition according to claim 1, **characterized in that** the polymer is:
• linear or branched and functionalised only at the ends, or
• linear or branched and comprising at least two binding groups (A) in the chain, or
• linear and carrying the binding groups (A) in side branches, or
• a dendrimer, or star-shaped, and having the binding groups (A) at the ends.

3. A composition according to claim 2, **characterized in that** the polymer is tri- or multifunctional, and preferably star-shaped or hyperbranched or branched, so that the composition has the form of a film after application onto keratin materials.

4. A composition according to any one of claims 1 to 3, **characterized in that** the polymer comprises at least one binding group (A₁) and at least one complementary binding group (A₂), each binding group (A₁) being liable to provide at least three H bonds with each complementary group (A₂), the binding groups (A₁) and (A₂) being selected by pair amongst the following pairs:
• bis(acylamino)pyridine/uracil or succinimide, or thymine, or glutarimide, or cyanuric acid, or the mixtures thereof,
• 2,4-diamino-s-triazine/uracil or succinimide, or thymine, or glutarimide, or cyanuric acid, or the mixtures thereof,
• monoacyl-2,4-diamino-s-triazine/uracil or succinimide, or thymine, or glutarimide, or cyanuric acid, or the mixtures thereof,
• melamine/uracil or succinimide, or thymine, or glutarimide, or cyanuric acid, or the mixtures thereof.

5. A composition according to any one of the preceding claims, **characterized in that** the -POL- polymeric backbone has a polymerisation degree ranging from 2 to 70,000, and preferably from 10 to 5000.

6. A composition according to any one of the preceding claims, **characterized in that** the -POL- polymeric backbone is selected amongst polydienes particularly hydrogenated polydienes, polyesters, polycarbonates, polyacetals, polyoxyalkylenes, polythioethers, perfluoropolyethers, polyolefins, polyorganosiloxanes, vinyl polymers, poly(meth)acrylics, cellulose derivatives, polysaccharide derivatives, hyperbranched or dendrimer compounds.

7. A composition according to any one of the preceding claims, **characterized in that** the polymer is a bifunctional POE/PPO copolymer having three ureidopyrimidone ends.

8. A composition according to any one of claims 1 to 6, **characterized in that** the polymer is a polydimethylsiloxane having two ureidopyrimidone ends.

9. A composition according to any one of claims 1 to 6, **characterized in that** the polymer is a polyester having two α,ω-ureidopyrimidone ends.

10. A composition according to any one of the preceding claims, **characterized in that** the composition has the form of an aqueous, alcoholic or hydroalcoholic solution or suspension, or of an oily solution or suspension, or a solution or a dispersion of the lotion or serum type, or an emulsion having a liquid or semi-liquid milk-like consistency, obtained through dispersion of a fatty phase in an aqueous phase (O/W) or vice versa (W/O), or a suspension or an emulsion with a soft consistency of the cream type (O/W) or (W/O), or an aqueous or anhydrous gel, or an ointment, a free or compacted powder to be used as such or to be incorporated into an excipient.

11. A cosmetic composition according to any one of preceding the claims, **characterized in that** it comprises at least one solvent selected amongst water, alcohols, polyols, short esters, hydrocarbon oils, silicone oils, fluorinated silicone oils and the mixtures thereof.

12. A composition according to any one of the preceding claims, **characterized in that** it comprises at least one builder selected amongst hydrophilic or lipophilic gels, hydrophilic or lipophilic active ingredients, preservatives, antioxidants, solvents, perfumes, fillers, hydrophilic screens, odour absorbers, neutralizing agents, other polymers other than those defined according to any one of the preceding claims, and emulsifiers.

13. A composition according to claim 12, **characterized in that** the active ingredients are selected amongst depigmenting agents, emollients, moisturizing agents, antiseborrheic agents, anti-acne agents, hair growth promoting agents, keratolytic and/or desquamative agents, antiwrinkle and tensor agents, anti-irritating agents, soothing agents, vitamins, screens, odour absorbers and the mixtures thereof.

14. A composition according to any one of the preceding claims, **characterized in that** it comprises a care composition, for keratin materials such as skin, lips and/or hairs, and/or in the form of a body hygiene composition such as a deodorant or a make-up removal product, or in the form of a make-up product for keratin materials, and/or in the form of a cleansing product, and/or in the form of a hair product.

15. A composition according to any one of the preceding claims, **characterized in that** it comprises at least one colouring material selected amongst lipophilic dyes, hydrophilic dyes, pigments, pearlescent materials and the mixtures thereof.

16. A composition according to any one of the preceding claims, **characterized in that** it has the form of a foundation cream, a blusher, a cheek or eyelid make-up, an eye-shadow concealer, an eyeliner, a temporary tattoo product for the body skin, a lip make-up product, such as a lipstick, a lip gloss, a lip pencil, or it has the form of a hair make-up product, or the form of a hair dye product.

17. A cosmetic care, making-up or treatment method for keratin materials in the human beings, comprising the application onto the keratin materials of a cosmetic composition, such as defined according to any one of the preceding claims.

18. A method for improving both the duration of at least one effect provided after deposition by a cosmetic composition and the adhesion of the composition applied on the keratin materials, and for also allowing for a quick, complete and selective make-up removal, consisting in adding to the composition an efficient amount of at least one linear or branched or cyclic polymer, or dendrimer, or hyperbranched polymer, comprising:
• a (POL) polymeric backbone comprising at least two repeating units, and
• at least two binding groups (A), fixed on the polymeric backbone and liable to create at least three H bonds with one or more linking binding groups, of an identical or different chemical nature, each coupling of two binding groups (A) involving at least three H bonds.

19. The use of an efficient amount of at least one linear, branched or cyclic polymer, or dendrimer, comprising:
• a -POL- polymeric backbone comprising at least two repeating units, and
• at least two binding groups (A), fixed on the polymeric backbone and liable to provide H bonds with one or more linking binding groups, of an identical or different chemical nature,
• each coupling of two binding groups involving at least three H bonds, in a cosmetic care and/or make-up composition for skin, lips or hairs, as an agent for improving the duration of an effect provided after deposition of the composition onto the skin, the lips or the hairs and/or for quickly and selectively removing the deposit of the composition.

## Patentansprüche

1. Kosmetische Zusammensetzung zur Pflege und/oder Behandlung und/oder zum Schminken von Keratinmaterialien, umfassend in einem physiologisch annehmbaren Medium eine wirksame Menge wenigstens eines linearen, verzweigten oder cyclischen oder dendrimeren Polymers, umfassend
• ein Polymergerüst -POL-, das wenigstens zwei Repetiereinheiten umfasst, und
• wenigstens zwei Verknüpfungsgruppen (A), die am Polymergerüst fixiert sind und fähig sind, Bindungen H mit einer Partnerverknüpfungsgruppe oder Partnerverknüpfungsgruppen mit identischer oder unterschiedlicher chemischer Natur zu entwickeln, wobei jede Paarung von zwei Verknüpfungsgruppen wenigstens drei Bindungen H einsetzt,
**dadurch gekennzeichnet, dass** die Verknüpfungsgruppen (A) ausgewählt sind unter: Adenin, Guanin, Cytidin, Thymin, Pterin, Ureidopyrimidon, Melamin, Cyanursäure, Maleimid, Phtalhydrazid, Isoguanin, Glycoluril, Uracil, Acylaminopyridin, vorzugsweise Monoacylaminopyridin und Bis(acylamino)-pyridin, Acylaminotriazin, vorzugsweise Monoacyl-2,4-diamino-s-triazin und Bisacyl-2,4-diamino-s-triazin, Pyridin/Phenol, Urazol, Glutarimid, Urazoylbenzoesäure, Succinimid, 2,4-Diamino-s-triazin, Ureidotriazin, Phtalhydrazid, Urazoylbenzoesäure.

2. Zusammensetzung nach Anspruch 1, **dadurch gekennzeichnet, dass** das Polymer ist:
• linear oder verzweigt und nur an den Enden funktionalisiert oder
• linear oder verzweigt und wenigstens zwei Verknüpfungsgruppen (A) in der Kette umfassend oder
• linear und die Verknüpfungsgruppen (A) als seitliche Verzweigungen tragend oder
• dendrimer oder als Stern und die Verknüpfungsgruppen (A) an den Enden tragend.

3. Zusammensetzung nach Anspruch 2, **dadurch gekennzeichnet, dass** das Polymer tri- oder multifunktionell ist und vorzugsweise als Stern oder hyperverzweigt oder verzweigt vorliegt, derart, dass die Zusammensetzung sich nach Auftragung auf die Keratinmaterialien in Form eines Films präsentiert.

4. Zusammensetzung nach einem der Ansprüche 1 bis 3, **dadurch gekennzeichnet, dass** das Polymer wenigstens eine Verknüpfungsgruppe (A₁) und wenigstens eine komplementäre Verknüpfungsgruppe (A₂) umfasst, wobei jede Verknüpfungsgruppe (A₁) mit jeder komplementären Gruppe (A₂) wenigstens drei Bindungen H entwickeln kann, wobei die Verknüpfungsgruppen (A₁) und (A₂) paarweise unter den folgenden Paaren ausgewählt sind:
• Bis(acylamino)pyridin/Uracil oder Succinimid oder Thymin oder Glutarimid oder Cyanursäure oder ihren Gemischen,
• 2,4-Diamino-s-triazin/Uracil oder Succinimid oder Thymin oder Glutarimid oder Cyanursäure oder ihren Gemischen,
• Monoacyl-2,4-diamino-s-triazin/Uracil oder Succinimid oder Thymin oder Glutarimid oder Cyanursäure oder ihren Gemischen,
• Melamin/Uracil oder Succinimid oder Thymin oder Glutarimid oder Cyanursäure oder ihren Gemischen.

5. Zusammensetzung nach einem der vorangehenden Ansprüche, **dadurch gekennzeichnet, dass** das Polymergerüst -POLeinen Polymerisationsgrad hat, der von 2 bis 70.000 und vorzugsweise von 10 bis 5.000 reicht.

6. Zusammensetzung nach einem der vorangehenden Ansprüche, **dadurch gekennzeichnet, dass** das Polymergerüst -POLausgewählt ist unter Polydienen, insbesondere hydrierten, Polyestern, Polycarbonaten, Polyacetalen, Polyoxyalkylenen, Polythioethern, Perfluorpolyethern, Polyolefinen, Polyorganosiloxanen, Vinylpolymeren, Poly(meth)acryl-Verbindungen, Cellulosederivaten, Polysaccharidderivaten, hyperverzweigten oder dendrimeren Polymeren.

7. Zusammensetzung nach einem der vorangehenden Ansprüche, **dadurch gekennzeichnet , dass** das Polymer ein bifunktionelles POE/PPO-Copolymer ist, das drei Ureidopyrimidon-Enden aufweist.

8. Zusammensetzung nach einem der Ansprüche 1 bis 6, **dadurch gekennzeichnet, dass** das Polymer ein Polydimethylsiloxan ist, das zwei Ureidopyrimidon-Enden aufweist.

9. Zusammensetzung nach einem der Ansprüche 1 bis 6, **dadurch gekennzeichnet, dass** das Polymer ein Polyester ist, der zwei α,ω-Ureidopyrimidon-Enden aufweist.

10. Zusammensetzung nach einem der vorangehenden Ansprüche, **dadurch gekennzeichnet, dass** sich die Zusammensetzung in Form einer wässrigen, alkoholischen oder wässrigalkoholischen Lösung oder Suspension oder in Form einer öligen Lösung oder Suspension oder in Form einer Lösung oder einer Dispersion des Lotion- oder Serumtyps, einer Emulsion mit flüssiger oder halbflüssiger Konsistenz des Milchtyps, erhalten durch Dispersion einer Fettphase in einer wässrigen Phase (Ö/W) oder umgekehrt (W/Ö), oder in Form einer Suspension oder Emulsion mit weicher Konsistenz des Typs Creme (Ö/W) oder (W/Ö) oder eines wässrigen oder wasserfreien Gels, in Form einer Salbe, eines freien Pulvers oder eines Kompaktpulvers, das frei zu verwenden ist oder in ein Exzipiens einzuarbeiten ist, präsentiert.

11. Kosmetische Zusammensetzung nach einem der vorangehenden Ansprüche, **dadurch gekennzeichnet, dass** sie wenigstens ein Lösungsmittel enthält, das ausgewählt ist aus Wasser, Alkoholen, Polyolen, kurzkettigen Estern, Kohlenwasserstoffölen, Silikonölen, fluorierten Silikonölen und ihren Mischungen.

12. Zusammensetzung nach einem der vorangehenden Ansprüche, **dadurch gekennzeichnet, dass** sie wenigstens ein Adjuvans umfasst, das ausgewählt ist aus hydrophilen oder lipophilen Geliermitteln, hydrophilen oder lipophilen Wirkstoffen, Konservierungsmitteln, Antioxidantien, Lösungsmitteln, Parfüms, Füllstoffen, hydrophilen Filtern, Geruchsabsorptionsmitteln, Neutralisiermitteln, anderen Polymeren als die, die in einem der vorangehenden Ansprüche definiert sind, und Emulgiermitteln.

13. Zusammensetzung nach Anspruch 12, **dadurch gekennzeichnet, dass** die Wirkstoffe ausgewählt sind unter Depigmentierungsmitteln, weichmachenden Mitteln, Hydratisierungsmitteln, Antiseborrhoika, Mitteln gegen Akne, Mitteln, die das Nachwachsen der Haare begünstigen, keratolytischen Mitteln und/oder Mitteln gegen Schuppen, Antifaltenmitteln und die Spannung erhöhenden Mitteln, Mitteln gegen Irritationen, beruhigenden Mitteln, Vitaminen, Filtern, Absorptionsmitteln für Geruch und ihren Gemischen.

14. Zusammensetzung nach einem der vorangehenden Ansprüche, **dadurch gekennzeichnet , dass** sie eine Pflegezusammensetzung für Keratinmaterialien, wie die Haut, die Lippen und/oder die Hautanhanggebilde, darstellt und/oder in Form einer Köperpflegezusammensetzung vorliegt, wie ein Deodorant-Produkt oder ein Reinigungsprodukt bzw. Abschminkprodukt, oder in Form eines Produkts zum Schminken der Keratinmaterialien und/oder in Form eines Reinigungsproduktes und/oder in Form eines Haarbehandlungsprodukts vorliegt.

15. Zusammensetzung nach einem der vorangehenden Ansprüche, **dadurch gekennzeichnet, dass** sie wenigstens ein färbendes Material enthält, das ausgewählt ist aus lipophilen Farbstoffen, hydrophilen Farbstoffen, Pigmenten, Perlmutt und ihren Mischungen.

16. Zusammensetzung nach einem der vorangehenden Ansprüche, **dadurch gekennzeichnet, dass** sie sich in Form einer Grundierung von Rouge, von Schminke für die Wangen, von Lidschatten, in Form eines Produktes gegen Aügenringe, in Form von Eyeliner, in Form eines Produktes für ein temporäres Tattoo der Körperhaut, in Form eines Schminkproduktes für die Lippen, wie Lippenstift, Lippenglanz, Lippenkonturenstift, oder in Form eines Schminkproduktes für die Hautanhanggebilde oder in Form eines Produktes zur Haarcoloration präsentiert.

17. Kosmetisches Verfahren zur Pflege, zum Schminken oder zur Behandlung der Keratinmaterialien von Menschen, umfassend das Auftragen einer kosmetischen Zusammensetzung, wie sie in einem der vorangehenden Ansprüche definiert ist, auf die Keratinmaterialien.

18. Verfahren, um gleichzeitig das Anhalten wenigstens einer Wirkung, die nach Auftragung von einer kosmetischen Zusammensetzung herbeigeführt wurde, und die Adhäsion der auf die Keratinmaterialien aufgetragenen Zusammensetzung zu verbessern und um auch ein schnelles, vollständiges und selektives Abschminken zu erlauben, das darin besteht, der Zusammensetzung eine wirksame Menge wenigstens eines linearen oder verzweigten oder cyclischen oder dendrimeren oder hyperverzweigten Polymers zuzusetzen, das umfasst:
• ein Polymergerüst (POL), das wenigstens zwei Repetiereinheiten umfasst, und
• wenigstens zwei Verknüpfungsgruppen (A), die am Polymergerüst fixiert sind und fähig sind, wenigstens drei Bindungen H mit einer Partnerverknüpfungsgruppe oder mehreren Partnerverknüpfungsgruppen mit identischer oder unterschiedlicher chemischer Natur zu entwickeln, wobei jede Paarung von zwei Verknüpfungsgruppen (A) wenigstens drei Bindungen H entwickelt.

19. Verwendung einer wirksamen Menge wenigstens eines linearen, verzweigten oder cyclischen oder dendrimeren Polymers, das umfasst:
• ein Polymergerüst -POL-, das wenigstens zwei Repetiereinheiten umfasst, und
• wenigstens zwei Verknüpfungsgruppen (A), die am Polymergerüst fixiert sind und fähig sind, Bindungen H mit einer Partnerverknüpfungsgruppe oder mehreren Partnerverknüpfungsgruppen mit identischer oder unterschiedlicher chemischer Natur zu entwickeln, wobei jede Paarung von zwei Verknüpfungsgruppen wenigstens drei Bindungen H einsetzt,
in einer kosmetischen Zusammensetzung zur Pflege und/oder zum Schminken der Haut, der Lippen oder der Hautanhanggebilde als Mittel, um das Anhalten einer nach Auftragung der Zusammensetzung auf die Haut, die Lippen oder die Hautanhanggebilde herbeigeführten Wirkung zu verbessern und/oder um die Auftragung der Zusammensetzung schnell und selektiv zu entfernen.
